# EUROPÄISCHE PATENTSCHRIFT

(11) **EP 2 585 059 B1**
(45) Veröffentlichungstag und Bekanntmachung des Hinweises auf die Patenterteilung: **09.08.2017**
(21) Anmeldenummer: 11736299.6
(22) Anmeldetag: 22.06.2011
(51) Int. Cl.: A61K 31/343, A61P 35/00

(54) **VERWENDUNG VON DIBENZOFURANONDERIVATEN ZUR INHIBIERUNG VON KINASEN**
USE OF DIBENZOFURANONE DERIVATIVES TO INHIBIT KINASES
UTILISATION DE DÉRIVÉS DE DIBENZOFURANONE POUR INHIBER DES KINASES

(30) Priorität: 25.06.2010 DE 102010025173
(43) Veröffentlichungstag der Anmeldung: 01.05.2013
(73) Patentinhaber: Jose, Joachim, 40589 Düsseldorf (DE)
(72) Erfinder: GÖTZ, Claudia, 66125 Saarbrücken (DE); GRATZ, Andreas, 40589 Düsseldorf (DE); KUCKLÄNDER, Uwe, 50259 Pulheim (DE); JOSE, Joachim, 40589 Düsseldorf (DE)
(74) Vertreter: Michalski Hüttermann & Partner Patentanwälte mbB
(86) Internationale Anmeldenummer: PCT/EP2011/060433
(87) Internationale Veröffentlichungsnummer: WO 2011/161151

(56) Entgegenhaltungen:
- WO-A1-91/16908
- US-A- 5 169 645
- UWE KUCKLÄNDER, HILDEGARD TÖBERICH: "Zur Umsetzung von 2-(Aminomethylen)cyclohexanon-Derivaten mit Dichlorchinonen", CHEMISCHE BERICHTE, Bd. 116, 1983, Seiten 152-158, XP000002659256,

## Beschreibung

Die vorliegende Erfindung betrifft ein Pharmazeutikum, welches als Wirksubstanz ein Dibenzofuranonderivat aufweist.

Die reversible Phosphorylierung von Proteinen ist ein wichtiger biologischer Regulationsmechanismus, der nahezu jeden Aspekt des Lebens auf zellulärer Ebene beeinflusst. Edmond H. Fischer und Edwin G. Krebs erhielten für ihre Pionierarbeit, die wesentlich zu dieser Erkenntnis beigetragen hat, 1992 den Nobelpreis für Medizin (Fischer, 1993; Krebs, 1993). Die Motivation zur Erforschung der Proteinphosphorylierung entwickelte sich zu Beginn der 1950er Jahre aus der Beobachtung, dass schnell proliferierendes Gewebe und insbesondere bestimmte Tumorzellen einen hohen Anteil an "Phosphoprotein" besaßen, welches darüber hinaus schnell umgesetzt wurde (Kennedy und Smith, 1954). Nachdem Phosphatasen als dephosphorylierende Enzyme bereits bekannt waren, suchte man nach Mediatoren der Proteinphosphorylierung. Die ersten Phosphoproteine, die dabei untersucht wurden, waren Nahrungseiweiße wie Phosvitin aus Eigelb und Casein aus Milch, da sie über Fällungsreaktionen leicht zu isolieren waren. Mit Casein identifizierten Burnett und Kennedy im Jahr 1954 die ersten "Protein Phosphokinasen" (Burnett und Kennedy, 1954). Hinter diesen "Caseinkinasen" verbargen sich nach heutiger Nomenklatur höchst wahrscheinlich die Proteinkinasen CK1 und CK2 (Pinna, 1994). Die physiologische Funktion dieser beiden Enzyme blieb aber noch unbekannt, weshalb dieser Entdeckung zunächst wenig Beachtung geschenkt wurde. Dies änderte sich bereits ein Jahr später mit der Entdeckung der Glycogen-Phosphorylase-b-Kinase (heute "Phosphorylase-Kinase" genannt) durch Fischer und Krebs (Krebs und Fischer, 1956). Die Identifizierung dieses regulatorischen Schlüsselenzyms des Glykogenstoffwechsels führte zu einer Beschleunigung der Proteinkinaseforschung.

Allerdings rückte ihr Fokus von der Krebsforschung weg und wurde zur Untersuchung der Proteinkinasen im Kontext metabolischer Prozesse verschoben. Seit dieser Zeit hat die Erforschung von Proteinkinasen kontinuierlich an Bedeutung gewonnen. Die Entdeckung und Untersuchung der cAMP-abhängigen Proteinkinase PKA (Walsh *et al.*, 1968), der cyclin-abhängigen Proteinkinasen, der mitogen-aktivierten Proteinkinasen und die Entdeckung einiger Rezeptor-Tyrosinkinasen (Fischer, 1993; Krebs, 1993) zeigte, dass Phosphorylierung ein generelles regulatorisches Prinzip darstellt. Die Konsequenzen sind dabei vielfältig. So kann Phosphorylierung beispielsweise die biologische Aktivität von Proteinen erhöhen oder reduzieren, diese für den Abbau markieren und ihre Stabilität beeinflussen. Darüber hinaus ermöglichen oder blockieren Phosphorylierungen und Dephosphorylierungen den Transport zwischen Zellkompartimenten oder die Interaktion zwischen Proteinen (Cohen, 2002a). Die wesentliche Reaktion, die von Proteinkinasen katalysiert wird, ist der Transfer des Phosphatrestes eines Donors, meist das γ-Phosphat von Adenosintriphosphat (ATP), auf das nukleophile Zentrum bestimmter proteingebundener Aminosäureseitenketten. In Eukaryoten kommen hauptsächlich Phosphorylierungen von Serin-, Threonin- und Tyrosinresten (*O*-Phosphate) vor (Ubersax und Ferrell, 2007). Darüber hinaus wurden auch Phosphate von Lysin-, Arginin- (Yan *et al.*, 1998) und Histidinresten (Klumpp und Krieglstein, 2005) gefunden (*N*-Phosphate), über deren Funktion zur Zeit aber noch wenig bekannt ist.

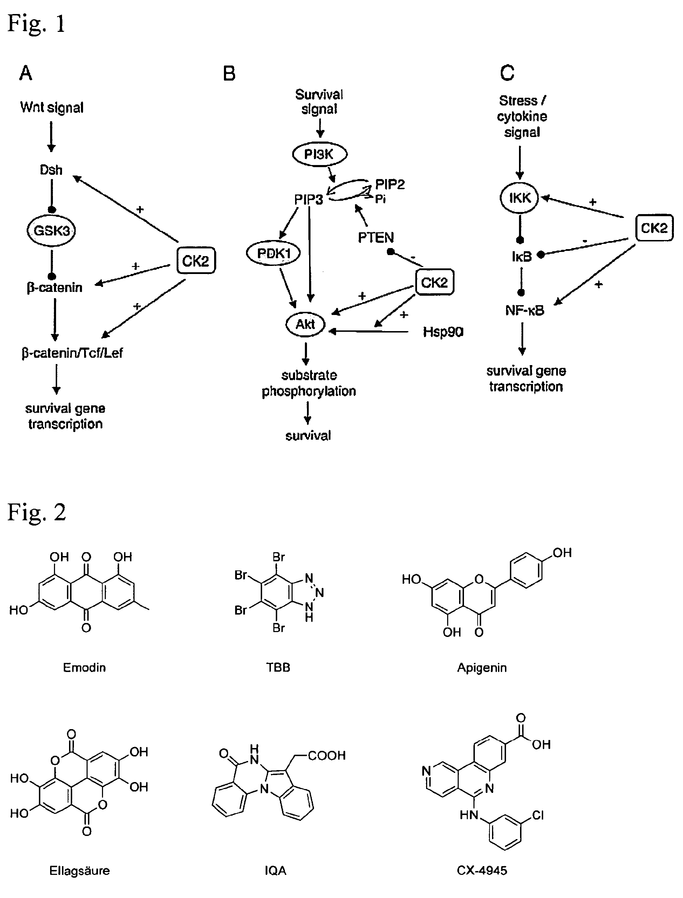

Im humanen Genom wurden 518 homologe Gene identifiziert, die potentiell für Proteinkinasen codieren. Für diese hat sich mittlerweile der Begriff humanes "Kinom" etabliert. Eine allgemein akzeptierte Einteilung des humanen Kinoms ist in Figur 1 dargestellt. Derzeit geht man davon aus, dass etwa ein Drittel aller Proteine der 20.000 bis 25.000 humanen Gene phosphoryliert ist. Die Gesamtheit dieser phosphorylierten Proteine wird als Phosphoproteom bezeichnet (Manning *et al.*, 2002). Da jedes Protein von verschiedenen Kinasen teilweise mehrfach phosphoryliert werden kann, errechnen sich für jede Proteinkinase im Durchschnitt etwa 20 Substrate (Cohen, 2002a). Obwohl die Primärstrukturen der Proteinkinasen teilweise stark differieren, ist der katalytische Kern der Proteinkinasen hochkonserviert und bildet eine bilobale Tertitärstruktur (Cheek *et al.*, 2002; Cheek *et al.*, 2005). Diese besteht aus einem N-terminalen Segment mit mehreren β-Faltblättern, einem "Gelenkbereich", der das aktive Zentrum beinhaltet, und einer α-Helixreichen C-terminalen Domäne.

Obwohl sie als eine der ersten Proteinkinasen entdeckt wurde, erregte die CK2 zunächst keine große Aufmerksamkeit. Im Nobelvortrag von Fischer und Krebs wurde sie beispielsweise nur in einem Nebensatz erwähnt (Fischer, 1993; Krebs, 1993). Folglich fand die Forschung zur bis heute nicht vollständig aufgeklärten physiologischen Funktion der Proteinkinase CK2 in eher kleinem Rahmen statt. Dies mag auch ein Grund sein, warum sie in den über 55 Jahren seit ihrer Entdeckung unterschiedlich benannt wurde.

Neben dem ursprünglichen Begriff Protein Phosphokinase von Burnett und Kennedy, wurde das Enzym in der Literatur z. B. als Nukleäre Proteinkinase NII (Thornburg und Lindell, 1977), eIF-2β-Kinase (DePaoli-Roach *et al.*, 1981), Troponin-T-Kinase (Villar-Palasi und Kumon, 1981), Caseinkinase TS (Deana *et al.*, 1978) oder auch Caseinkinase II (Prowald *et al.*, 1984) bezeichnet. Auf einer Konferenz führender CK2-Forscher im Jahre 1994 wurde schließlich beschlossen, Proteinkinase CK2 oder das Akronym CK2 als Bezeichnungen für diese Proteinkinase zu verwenden (Ahmed *et al.*, 1994).

Die humane Serin-/Threonin-Proteinkinase CK2 bildet eine heterotetramere Quartärstruktur, für die sich in der Literatur die Bezeichnung Holoenzym durchgesetzt hat. Es liegt als Komplex aus katalytischer Untereinheit (CK2α) und nicht-katalytischer Untereinheit (CK2β) in einer α₂β₂-Stöchiometrie vor. Neben der am häufigsten vorkommenden, 44 kDa großen katalytischen α-Untereinheit gibt es zwei weitere Isoformen dieser Untereinheit (α', 38 kDa (Lozeman *et al.*, 1990) und α", 44 kDa (Shi *et al.*, 2001)). Diese Isoformen zeigen ebenfalls katalytische Aktivität, sind aber weniger gut untersucht (Litchfield *et al.*, 2001). Die 26 kDa große β-Untereinheit besitzt eine Zinkfingerdomäne, die eine Dimerisierung zweier β-Monomere ermöglicht. Das Dimer bildet den Ankerpunkt für die Oligomerisierung des Enzyms (Graham und Litchfield, 2000). An der β-dimeren Kernstruktur binden zwei α-Monomere in der Weise, dass sie im Komplex keinen direkten Kontakt miteinander haben (Niefind *et al.*, 2001). Diese Oligomerisierung findet spontan statt und ist sehr stabil. Es gibt aber auch Hinweise, dass die CK2 *in vivo* nicht ausschließlich als tetrameres Holoenzym vorliegt. Seinen isolierten Untereinheiten könnte auch eine physiologische Funktion zukommen (Faust und Montenarh, 2000; Bibby und Litchfield, 2005). Weiterhin konnten ringförmige oder filamentöse Aggregate mehrerer CK2-Holoenzyme *in vitro* nachgewiesen werden (Valero *et al.*, 1995; Niefind und Issinger, 2005).

Die Klassifizierung der CK2 beruht auf der katalytisch aktiven α-Untereinheit. Aufgrund ihrer Sequenzhomologie wird sie der Familie der CMGC-Proteinkinasen zugeordnet (siehe Figur 1). Diese umfasst die Cyclin-abhängigen Kinasen (CDK), die Mitogen-aktivierten Proteinkinasen (MAP-Kinasen), die Glycogensynthase-Kinase 3-ähnlichen Kinasen (GSK3) und CDK-ähnlichen Kinasen (CLK). Die nächsten Verwandten der CK2 in dieser Familie sind die MAP-Kinasen, die CDKs und die GSK3 (Manning *et al.*, 2002). Die Sequenzidentität der CK2α zu diesen Enzymen liegt bei bis zu 35 %.

Im Gegensatz zu anderen Vertretern der Proteinkinasen weist die CK2 einige auffällige Besonderheiten auf. In den letzten Jahren ist die Anzahl der experimentell bestätigten Substrate nahezu exponentiell gestiegen und liegt mittlerweile bei deutlich über 300 (Meggio und Pinna, 2003; Salvi *et al.*, 2009). Diese als "Pleiotropie" bezeichnete Eigenschaft ist für Proteinkinasen äußerst ungewöhnlich und spiegelt sich in den bereits erwähnten, multiplen Bezeichnungen für dieses Enzym wider.

Die meisten Proteinkinasen zeigen keine oder lediglich eine niedrige basale Aktivität. Ihre Aktivierung ist meist streng reguliert und erfolgt durch einen Reiz, einen Effektor oder einen physiologischen Status (Blume-Jensen und Hunter, 2001). Im Gegensatz dazu besitzt die CK2 eine hohe zelluläre Aktivität (Pinna, 1990). Sowohl die isolierte CK2α, als auch das tetramere Holoenzym sind konstitutiv aktiv. Bislang ist auf Proteinebene kein für Kinasen typischer Mechanismus bekannt, der die CK2-Aktivität reguliert. Stattdessen hat sich in den letzten Jahren ein mehrschichtiges Modell für die Regulation der CK2 etabliert, das den Großteil der empirischen Erkenntnisse über die komplexe Regulation der CK2 einbezieht und erste Erklärungsversuche bietet (Filhol und Cochet, 2009). Demnach ist das Niveau der CK2-Aktivität einer spatiotemporalen Regulation unterworfen. Beispielsweise kann sich die Substratspezifität der CK2α nach Tetramerisierung zum Holoenzym oder in höheren Assoziationsformen ändern (Meggio *et al.*, 1992; Valero *et al.*, 1995; Salvi *et al.*, 2006). Dieser Effekt wurde auch nach intermolekularer Assoziationen mit diversen Substraten oder Bindepartnern beobachtet (Olsten und Litchfield, 2004; Olsten *et al.*, 2005). Auf diese Weise könnte die Aktivität, bezogen auf ein bestimmtes Substrat, durch allosterische Effektoren moduliert werden. Außerdem findet eine dynamische Lokalisierung der CK2, bzw. ihrer Untereinheiten zwischen verschiedenen subzellulären Kompartimenten statt, vorwiegend zwischen Kern und Zytoplasma (Faust und Montenarh, 2000). So kann die Aktivität der CK2 in bestimmten Kompartimenten variiert werden, während die Gesamtaktivität der Zelle konstant bleibt. Ein weiteres atypisches Merkmal der CK2 ist ihre duale Cosubstratspezifität für ATP und GTP (Niefind *et al.*, 1999). Beide Moleküle können nahezu gleichwertig als Phosphatdonoren genutzt werden. Mit Ausnahme der Src-Kinase (Graziani *et al.*, 1983) und der EGF-Rezeptorkinase (Carpenter *et al.*, 1979) ist dies keiner anderen Proteinkinase möglich. Neue Untersuchungen zeigen außerdem, dass die CK2 unter gewissen Umständen neben Serin- und Threonin- auch Tyrosinreste phosphorylieren kann. Neben der dualen Cosubstrat- könnte sie also auch eine duale Substratspezifität besitzen (Vilk *et al.*, 2008).

Generell ist die CK2α acidophil, d. h. die Sequenzumgebung des Substrats, in der sich der zu phosphorylierende Rest (n) befindet, muss saure Aminosäuren aufweisen. Eine minimale Konsensussequenz für Substrate lautet (n)-X-X-(D/E/Sp/Tp) (Meggio und Pinna, 2003). In Position (n+3) scheint eine negativ geladene Aminosäureseitenkette essentiell für die Substraterkennung zu sein. Diese kann auch durch ein phosphoryliertes Serin (Sp) oder Threonin (Tp) dargestellt werden. Weitere negative Ladungen, v. a. in Position (n+1) sind förderlich. Als Modellsubstrat zur Untersuchung der CK2-Aktivität hat sich in mehreren Arbeitsgruppen das Peptid mit der Sequenz RRRDDDSDDD durchgesetzt (Kuenzel *et al.*, 1987; Olsen *et al.*, 2006; Schneider *et al.*, 2010), das auch in dieser Arbeit Verwendung findet.

Zur β-Untereinheit der CK2 findet sich, ganz im Gegensatz zur α-Untereinheit, kein Homolog im humanen Proteom. Sie zeigt aber eine extrem hohe Konservierung zwischen den verschiedenen Spezies (Allende und Allende, 1995), was eine wichtige funktionelle Rolle für dieses Protein impliziert. In der Literatur wird sie bisweilen als "regulatorische Untereinheit" beschrieben, da sie die Substratspezifität beeinflussen und so die Aktivität des CK2-Holoenzyms modulieren kann (Meggio *et al.*, 1992; Tiganis *et al.*, 1993; Salvi *et al.*, 2006). Andererseits wird aus der Kristallstruktur des Holoenzyms ersichtlich, dass CK2β vermutlich keinen direkten Einfluss auf die katalytischen Schlüsselstrukturen der CK2α hat (Niefind *et al.*, 2001). Eine Rolle als "Kontaktvermittler" für Substrate scheint für CK2β denkbar. Unstrittig ist bislang, dass mit der CK2β-induzierten Oligomerisierung eine drastisch erhöhte Stabilität und eine verminderte Proteasesensitivität einhergeht (Meggio *et al.*, 1992).

Es sprechen mehrere Indizien für eine wichtige physiologische Rolle der CK2. Ihre Aminosäuresequenz ist über Speziesgrenzen hinweg hochkonserviert. Darüber hinaus kann CK2-Aktivität nahezu in allen Geweben, Zelltypen und in vielen Zellkompartimenten nachgewiesen werden. Auch die große Anzahl an Interaktionspartnern, sowie ihre konstitutive Aktivität legen eine wichtige Rolle nahe. Im Folgenden werden kongruente Erkenntnisse auf Schlüsselfunktionen der CK2 im zellulären Kontext hervorgehoben. Auf Wirkungen der CK2 innerhalb verschiedener Signaltransduktionswege und deren Konsequenzen wird weiter unten eingegangen.

Die CK2 unterscheidet sich funktionell von den meisten anderen Proteinkinasen, die ihre Aufgabe an einer spezifischen Stelle innerhalb eines streng hierarchisch gegliederten "vertikalen" Signaltransduktionsweges in binärem Modus ausüben. Meggio und Pinna formulierten die Einschätzung, dass die Informationsübertragung durch die CK2 vielmehr auf lateraler Ebene stattfindet und mehrere Signalwege verknüpft (Meggio und Pinna, 2003). Der CK2 könnte so eine grundlegende Bedeutung für basale Zellfunktionen zukommen. Ihr ubiquitäres Vorkommen, ihre hohe konstitutive Aktivität und ihre über 300 Substrate legen die Vermutung nahe, dass die CK2 maßgeblich daran beteiligt ist, das Phosphoproteom auf einem lebenswichtigen Niveau zu halten (Barz *et al.*, 2003). Metaphorisch ausgedrückt kommt der CK2 damit eher die Rolle des teamorientierten Spielmachers als die des hochspezialisierten Torjägers zu.

Im Gegensatz zu dieser eher globalen, recht unspezifischen Aufgabe häufen sich aktuell Ergebnisse, die der CK2 eine Rolle im Biorhythmus zuweisen. So wurde bei *Drosophila melanogaster* eine hohe Expression von CK2α im Zytoplasma von Neuronen detektiert, die eine Schrittmacherfunktion für die innere Uhr besitzen. Heterozygot CK2α-defiziente Fruchtfliegen haben einen sogenannten "timekeeper"-Phänotyp: Sie zeigen eine um ca. 1 h verlängerte Rhythmik (Lin *et al.*, 2002). Ergebnisse ähnlicher *in* vivo-Studien in Pflanzen (*Arabidopsis*) (Sugano *et al.*, 1999), Pilzen (*Neurospora*) (Yang *et al.*, 2002), Mäusen (Tsuchiya *et al.*, 2009) und in humanen Zellen (Maier *et al.*, 2009) zeigen ebenfalls einen direkten Zusammenhang von CK2-Aktivität und Biorhythmus. Die CK2 scheint ein wichtiges, konserviertes Element zu sein, das die Steuerung des Biorhythmus in diesen drei phylogenetischen Reichen verbindet.

Die lebenswichtige Bedeutung der CK2 für diverse Organismen wurde durch Stilllegung der Expression der CK2-Gene nachgewiesen. Bei Mäusen führte sowohl ein "knockout" von CK2β (Buchou *et al.*, 2003), als auch von CK2α (Lou *et al.*, 2008) zu embryonaler Letalität. Im letzten Fall starben die Mäuse in der Embryogenese mit Entwicklungsdefekten in Herz und Neuralröhre (Seldin *et al.*, 2008). Die Deletion von CK2α' in männlichen Mäusen konnte während der Embryonalentwicklung von CK2α kompensiert werden, führte später aber zu defekter Spermatogenese und Unfruchtbarkeit (Xu *et al.*, 1999b; Escalier *et al.*, 2003). Deletionsexperimente mit *Saccharomyces cerevisiae* (Glover, 1998) und humanen Fibroblasten (Lorenz *et al.*, 1994) bestätigten die lebenswichtige Funktion der CK2. Abwesenheit der CK2-Aktivität führte in diesen Experimenten zum Zellzyklusarrest. Kürzlich wurde gezeigt, dass die Phosphorylierung von eIF5 ("eukaryotic translation initiation factor 5") durch CK2 ein essentielles Ereignis für das Fortschreiten des Zellzyklus darstellt (Homma und Homma, 2008). Bei dieser Untersuchung konnte die funktionelle Aktivierung der CK2 mit einer Translokation vom Zytoplasma in den Zellkern korreliert werden. Diese Erkenntnisse vermuten, dass der CK2 eine tragende Rolle in der Regulation des Zellzyklus und der Zellproliferation zukommt.

Andere Hinweise deuten auf eine Schlüsselfunktion der CK2 als negativer Regulator der Apoptose hin. In der Apoptose spielen spezifische proteolytische Ereignisse eine zentrale Rolle, die von Caspasen ausgeführt werden. Für einige Caspase-Substrate wurde gezeigt, dass deren Empfindlichkeit für Caspase-vermittelte Degradation nach Phosphorylierung durch die CK2 stark herabgesetzt ist (Desagher *et al.*, 2001; Ruzzene *et al.*, 2002). Verantwortlich dafür könnte eine ähnliche Konsensussequenz von Caspase- und CK2-Substraten sein (Litchfield, 2003). Zusätzlich kann die CK2 die Aktivität von Caspasen auch direkt hemmen. Eine Phosphorylierung der murinen Caspase-9 durch CK2 beispielsweise schützt vor deren Abbau durch Caspase-8 (McDonnell *et al.*, 2008). Des Weiteren verhindert eine CK2-vermittelte Phosphorylierung der Caspase-2 deren Dimerisierung und verhindert so ihre Aktivierung (Shin *et al.*, 2005). Die Phosphorylierung von ARC ("apoptose repressor with caspase recruitment domain") durch CK2 ist Voraussetzung für seine effektive Hemmung der Caspase-8 (Li *et al.*, 2002). Die postulierte Korrelation der CK2-Aktivität und der Unterdrückung der Apoptose konnte durch Zellkulturexperimente belegt werden, in denen die Aktivität der CK2 auf ein leicht erniedrigtes Niveau gebracht wurde (Unger *et al.*, 2004; Wang *et al.*, 2005). Diese Zellen waren deutlich sensitiver gegenüber Apoptoseinduzierenden Reizen als Zellen mit normalem Niveau an CK2-Aktivität. Umgekehrt machte eine Überexpression der CK2 die Zellen resistenter gegen diese Reize (Ahmad *et al.*, 2008).

Die CK2 wurde mit einer Reihe von Erkrankungen in direkte oder mittelbare Verbindung gebracht, von denen einige in Tabelle 1 aufgelistet sind. Dabei wurden verschiedene Arten der Dysregulation essentieller Informationsübertragung nachgewiesen. Im Falle viraler Erkrankungen wird die CK2-Aktivität teilweise parasitär von viralen Proteinen genutzt (Filhol und Cochet, 2009). Die enorme Anzahl der gefundenen Implikationen dieses Enzyms basiert vermutlich auf der erläuterten Vielzahl an Substraten und den daraus resultierenden pleiotropen Effekten. Erkenntnisse zu der Rolle der CK2 in diesen und anderen Krankheiten werden in einem Übersichtsartikel von Guerra und Issinger (2008) diskutiert. Die meisten und am besten erforschten Hinweise auf eine pathologische Wirkung dysregulierter CK2 gibt es jedoch im Kontext von Krebserkrankungen. Dieser Zusammenhang wird im Folgenden aus mehreren Perspektiven beleuchtet, die sowohl rationale als auch empirische Fakten einbeziehen.

**Tabelle 1: Übersicht humaner nicht-onkologischer Erkrankungen, bei denen es Hinweise auf eine Beteiligung der CK2 gibt.**

| **Krankheitsgebiet** | **Beispiele / Erreger** | **Referenz** |
|---|---|---|
| Neurodegeneration | Alzheimer | (Blanquet, 2000) |
| | Parkinson | (Ryu *et al.*, 2008) |
| | Spongioforme Enzephalopathie | (Chen *et al.*, 2008) |
| Entzündungsprozesse | Autoimmunerkrankungen (z.B. RA) | (Shimoyama *et al.*, 2001) |
| | Glomerulonephritis | (Yamada *et al.*, 2005) |
| | Mukoviszidose | (Pagano *et al.*, 2010) |
| Protozoeninfektionen | Chagas (*Trypanosoma cruzi*) | (Augustine *et al.*, 2006) |
| | Malaria (*Plasmodium* | (Hora *et al.*, 2009) |
| | *falciparum*) | |
| | Toxoplasmose (*Toxoplasma gondii*) | (Delorme *et al.*, 2003) |
| Virale Infektion | EBV | (Medina-Palazon *et al.*, 2007) |
| | HIV | (Caples *et al.*, 2006) |
| | HPV | (Chien *et al.*, 2000) |
| Vaskuläre Erkrankungen | Arteriosklerose | (Harvey *et al.*, 2007) |

Viele Jahre der Krebsforschung auf medizinisch-anatomischer, zellulärer, biochemischer und molekulargenetischer Ebene haben eine unermessliche Anhäufung von Fakten und Einschätzungen zur Entstehung der Krankheit hervorgebracht, die heutzutage in ihrer Komplexität nicht mehr vollständig zu erfassen sind. Folglich muss versucht werden, die Onkogenese mit einigen Kernprinzipien zu beschreiben, die den meisten Krebserkrankungen zugrunde liegen. Allgemein wird davon ausgegangen, dass die Entstehung von Krebs ein mehrstufiger Prozess ist, der zum Erwerb einiger Defekte und Fähigkeiten führt ("cancer acquired capabilities"), die zusammen eine onkogene Transformation bewirken. Neben der modifizierten Reaktion auf äußere Reize der Mikroumgebung muss es zum Verlust zellautonomer Kontrollmechanismen kommen. Von Hanahan und Weinberg wurden Anfang des Jahrtausends sechs Hauptmerkmale ("hallmarks") für die Manifestation eines Tumors formuliert: 1) Autarkie bezüglich Wachstumssignalen; 2) Resistenz gegenüber wachstumshemmenden Signalen; 3) Unterdrückung von Apoptose; 4) Unbegrenztes Replikationspotential; 5) Nachhaltige Angiogenese und 6) Invasives Wachstum und Metastasierung (Hanahan und Weinberg, 2000). Viele dieser Fähigkeiten werden durch ein erhöhtes CK2-Niveau begünstigt. Wie bereits erwähnt, stört eine gesteigerte CK2-Aktivität die Kontrolle des Zellzyklus und des Zellwachstums und hat anti-apoptotische Wirkungen. In der parasitären Rindererkrankung Theileriose (Ostafrika-Fieber) wurde mit diesen Erkenntnissen übereinstimmend eine erhöhte CK2-Aktivität festgestellt, die mit einer starken Proliferation von Lymphozyten einhergeht (ole-MoiYoi, 1995). Weiterhin ist ein Zusammenhang zwischen CK2-Aktivität und Angiogenese gefunden worden (Mottet *et al.*, 2005; Kramerov *et al.*, 2008).

Neben diesen direkten Verknüpfungen von Krebsmerkmalen und CK2-Aktivität gibt es weitere Verknüpfungen, deren kausale Zusammenhänge teilweise indirekt und weniger eindeutig sind. Beispielsweise wird ein gestörter Biorhythmus, dessen Regulation die CK2 beeinflussen kann, mit Krebserkrankungen in Verbindung gebracht (Fu und Lee, 2003). Weiterhin wurde die CK2 kürzlich als ein wichtiger Regulator von Entzündungsreaktionen postuliert (Singh und Ramji, 2008), was auf ihre weiter unten beschriebene Rolle im NF-κB-Weg zurückzuführen sein könnte (Figur 2 C). Die Bedeutung von Entzündungsprozessen in der Onkogenese wird schon länger diskutiert (Mantovani *et al.*, 2008). Diese Beispiele zeigen, dass viele der durch CK2-Aktivität beeinflusste zelluläre Merkmale mit Hauptmerkmalen der Onkogenese übereinstimmen.

Mehrere Untersuchungen legen eine direkte Verknüpfung von CK2 und krebsrelevanten zellulären Prozessen nahe. So interagiert die CK2 über verschiedene Mechanismen mit mehreren Proteinen und Signaltransduktionswegen, die direkte Auswirkungen auf die Onkogenese haben. Dadurch lassen sich einige der von CK2 beeinflussten Krebsmerkmale auf molekularer Ebene erklären.

Der Wnt-Signalweg (Figur 2 A) treibt durch Aktivierung von Transkriptionsfaktoren die Embryogenese, die Zelldifferenzierung und die Zellteilung voran. Zielgene dieses Signalwegs gehören zu den am häufigsten mutierten Genen in menschlichen Tumoren. Der klassische Wnt-Signalweg wird durch einen extrazellulären Wnt-Faktor aktiviert, der an einen Transmembranrezeptor der Fizzeld Familie (Fz) bindet. Danach kommt es zu einer Dimerisierung von Fz mit dem Corezeptor LRP und einer Aktivierung des zytoplasmatischen Proteins Dishevelled (Dsh). Die Phosphorylierung von Dsh wird durch CK2 vermittelt und ist wichtig für dessen Hemmwirkung auf die Glykogensynthase-Kinase3β (GSK3β). Durch eine Hemmung der GSK3β kann β-Catenin das Wnt-Signal bis in den Zellkern weiterleiten, anstatt nach GSK3β-Phosphorylierung abgebaut zu werden (Willert *et al.*, 1997). Inzwischen wird davon ausgegangen, dass β-Catenin und sein Rezeptor, der Transkriptionsfaktor TCF/LEF, selbst Substrate der CK2 sind und CK2-vermittelte Phosphorylierung ihre Aktivität fördern (Wang und Jones, 2006). Durch Verstärkung des Wnt-Signals wird die Expressionsstärke der Wnt-Zielgene gesteigert. Unter diesen Zielgenen befinden sich auch mehrere Proto-Onkogene. Eine gesteigerte CK2-Aktivität könnte über die geschilderten lateralen Mechanismen zu einer Verstärkung des Wnt-Signalwegs führen und über eine vermehrte Expression von Proto-Onkogenen die Onkogenese begünstigen (Seldin *et al.*, 2005; Dominguez *et al.*, 2009).

Weitere Substrate der CK2 finden sich im PI3K/Akt-Weg (Figur 2 B). Allgemein hat die Aktivierung dieses Signalwegs eine anti-apoptotische Wirkung (Sale und Sale, 2008; Chalhoub und Baker, 2009). PTEN ("phosphatase and tensin homolog deleted on chromosome 10") ist ein Tumorsupressor und eines der CK2-Substrate, die in diesem hierarchischen Weg auf einer hohen Ebene angesiedelt sind. In aktiver Form stellt PTEN einen negativen Regulator dieses Wegs da. Phosphorylierung durch die CK2 hat sowohl negative Auswirkungen auf seine Stabilität, als auch auf seine Phosphataseaktivität (Torres und Pulido, 2001). Eine zweite zentrale Komponente dieses Signalwegs ist Akt, die auch Protein Kinase B genannt wird. Eine CK2-vermittelte Phosphorylierung potenziert die Aktivität von Akt (Di Maira *et al.*, 2005). Sie unterstützt vermutlich die Komplexbildung von Akt mit dem Chaperon Hsp90, was zu einer Stabilisierung des aktiven Status von Akt führt (Sato *et al.*, 2000). Beide Effekte der CK2, die Abschwächung von PTEN und die Aktivierung von Akt, führen zu einer Verstärkung des PI3K/Akt-Wegs. Zusammen mit der beschriebenen Hemmung von Caspasen kommt der CK2 so eine globale Rolle als antiapoptotischem Regulator zu, dessen dysregulierte Aktivität onkogenes Potential entwickelt.

Ein weiterer Signalübertragungsweg, der durch CK2-Aktivität moduliert werden kann, ist der NF-κB-Weg (Figur 2 C). Neben seiner Hauptaufgabe in Entzündungsprozessen wurde dieser Weg auch mit pro-proliferativer und anti-apoptotischer Aktivität sowie Zelltransformation in Verbindung gebracht. Dadurch beeinflusst er einige der angesprochenen Hauptkennzeichen der Onkogenese (Ravi und Bedi, 2004). CK2 verstärkt auch in diesem Fall den Signalweg über verschiedene Mechanismen durch laterale Interaktion mit IKK, IκB und NF-κB (Dominguez *et al.*, 2009).

Im Kontext dieser kaskadischen Signalweiterleitungen zeigt sich abermals, dass CK2 keine eindeutig in eine spezifische physiologische Funktion eingebundene Aufgabe besitzt. Die beschriebenen Signalwege beeinflussen hauptsächlich Zelldifferenzierung, -proliferation und -überleben, also genau die zellulären Eigenschaften, deren fehlerhafte Ausprägungen in der Onkogenese zu beobachten sind. Der Einfluss der CK2 ist hier lateral, führt also zu einer Modulation des "vertikalen" Informationsflusses und potenziert in allen Fällen den ursprünglichen Reiz (Figur 2).

CK2 stellt somit eine Art Hauptregulator und gleichzeitig ein Bindeglied essentieller Zellfunktionen dar. Es ist deshalb wenig verwunderlich, dass eine Änderung seiner zellulären Gesamtaktivität tiefgreifende Auswirkungen hat. Tatsächlich wurde in Untersuchungen vieler Krebsarten eine Korrelation zwischen gesteigerter CK2-Aktivität und Induktion, bzw. Verstärkung der beschriebenen Signalwege festgestellt (Seldin *et al.*, 2005; Guerra, 2006; Di Maira *et al.*, 2009; Dominguez *et al.*, 2009).

Die meisten Substrate der CK2 sind nicht in solche Signalwege eingebunden. Einige sind aber direkt an der Krebsentstehung beteiligt. Der Transkriptionsfaktor p53 wird als "Wächter des Genoms" bezeichnet (Lane, 1992). Er vermittelt nach Induktion durch Zellstress die Biosynthese von Proteinen, die den Zellzyklus stoppen, die Angiogenese hemmen, die Apoptose induzieren oder zum DNA-Reparatursystem der Zelle gehören. Die p53-vermittelten Effekte wirken insgesamt einer Onkogenese entgegen und p53 wird als eines der wichtigsten Tumorsupressorproteine angesehen (Brown *et al.*, 2009). Tatsächlich scheint die Inhibition von p53 ein Schlüsselfaktor von Krebs zu sein, da in über 50 % der humanen Tumoren eine Veränderung des p53-Gens vorliegt, die seine Wirkung abschwächt oder ganz unterdrückt (Montenarh, 1997). Neben einer direkten Phosphorylierung von p53 durch CK2 wurden nicht-enzymatische Interaktionen von CK2α und CK2β mit p53 gefunden, welche die Funktion von p53 und der CK2 beeinflussen. Daneben interagiert die CK2 auch mit p53-regulierenden Enzymen, wie z. B. Mdm2 (Allende-Vega *et al.*, 2005). Alle diese CK2-vermittelten Effekte hemmen die Aktivität von p53. Ein weiteres krebsrelevantes CK2-Substrat ist der Tumorsupressor PML ("promyelocytic leukemia tumor suppressor")(Salomoni und Pandolfi, 2002). Ein Ubiquitin-vermittelter Abbau von PML in Krebszellen ist die direkte Folge einer Phosphorylierung durch gesteigerte CK2-Aktivität (Scaglioni *et al.*, 2008).

So unterschiedlich die Mechanismen der Interaktion zwischen der CK2 und ihren Substraten auch sind, das Resultat ist in den beschriebenen Fällen immer dasselbe: In gesunden Zellen ist die Aktivität der CK2 wichtig für die normale Zellfunktion. Ihre Erhöhung führt allerdings zu einer Schwächung zellulärer Abwehrmechanismen gegen die Entstehung von Krebs und begünstigt Zustände, die eine Onkogenese fördern.

Untersuchungen des CK2-Niveaus in Krebszellen liefern die bisher eindeutigsten Hinweise auf eine Rolle der CK2 im Krebsgeschehen. In jedem Krebsgewebe, das bislang auf CK2 untersucht wurde, fand man ein verstärkte Expression der CK2 oder eine erhöhte CK2-Aktivität verglichen mit entsprechendem gesundem Gewebe (Trembley *et al.*, 2009). Zum Beispiel wurde in humanen Mammakarzinomen eine CK2-Aktivität festgestellt, die im Durchschnitt um den Faktor 10 höher war als in gesundem Brustgewebe (Landesman-Bollag *et al.*, 2001a; Landesman-Bollag *et al.*, 2001b). Auch die CK2α-Konzentration in diesen Tumoren war deutlich erhöht. In einer anderen Studie wurde die Aktivität und die Expression von CK2 in histologisch unterschiedlichen Nierentumoren mit ipsilateralem Normalgewebe verglichen (Siemer *et al.*, 1996). Bei der quantitativen Untersuchung der Untereinheiten wurde in allen Nierentumoren ein Anstieg sowohl der α-, als auch der β-Untereinheit detektiert. Die Aktivität der CK2 war in den unterschiedlichen Nierentumoren durchschnittlich um den Faktor 2 erhöht. Zu der größten Aktivitätszunahme von 375 % kam es beim malignen Nephroblastom. Beim benignen Onkozytom betrug die Zunahme dagegen lediglich 59 %. Außerdem wurden in diesen Tumoren Hinweise auf eine heterogene Expressionsstärke der beiden CK2-Untereinheiten im Vergleich zum Normalgewebe gefunden (Stalter *et al.*, 1994), die auch in anderen Untersuchungen nachgewiesen werden konnten (Faust *et al.*, 1999). Ein erhöhtes CK2-Niveau wurde auch in einer Vielzahl weiterer Krebserkrankungen festgestellt (Guerra und Issinger, 2008), u. a. bei Kolonkarzinomen (Münstermann *et al.*, 1990), bei Leukämie (Roig *et al.*, 1999), in Tumoren der Lunge (Daya-Makin *et al.*, 1994), des Endometriums (Llobet *et al.*, 2008; Pallares *et al.*, 2009), sowie der Prostata (Yenice *et al.*, 1994; Laramas *et al.*, 2007). Verschiedene Studien deuten darüber hinaus auf einen direkten Zusammenhang zwischen dem Ausmaß der CK2-Aktivitätssteigerung und der Malignität der Krebserkrankung hin (Tawfic *et al.*, 2001; Unger *et al.*, 2004). Aufgrund dieser Erkenntnisse wird CK2α als prognostischer Marker für diverse Krebsarten diskutiert (Kim *et al.*, 2007; Laramas *et al.*, 2007).

Bislang ist noch unklar, wie und auf welcher Ebene der neoplastischen Transformation die Steigerung der CK2-Aktivität einsetzt. Sie scheint allerdings keine Folge der starken Proliferation von Krebszellen zu sein, da bereits in präkanzeröser Dysplasie erhöhte CK2-Aktivität festgestellt werden konnte (Faust *et al.*, 1999). Anders als bei krebsrelevanten Onkogenen oder Tumor-Supressorgenen wurde interessanterweise bislang keine Mutation des CK2-Gens gefunden, die zu einer Hyperaktivität des Enzyms führt. Hauptmechanismus der Aktivitätssteigerung der CK2 in Tumorzellen scheint demnach die Erhöhung der Enzymmenge aufgrund einer verstärkten Expression der CK2-Gene zu sein. Daneben könnte auch die bislang unvollständig verstandene Regulation der CK2 eine Rolle spielen (Guerra und Issinger, 1999, 2008).

Neben diesen deskriptiven Studien wurde in komplementären Untersuchungen die Expression der CK2 in Modellsystemen künstlich erhöht, um die pathophysiologischen Konsequenzen zu beobachten. Transgene Mäuse mit gesteigertem lymphozytärem CK2-Expressionsniveau zeigten eine deutlich erhöhte Inzidenz für Leukämie. Sie entwickelten ab dem 6. Lebensmonat Lymphome mit einer Inzidenz von 6-15 % pro Jahr (Seldin und Leder, 1995; Kelliher *et al.*, 1996). Wenn zusätzlich zu CK2α ein Onkogen, z. B. *c-myc* oder *TAL-1*, überexprimiert wird, verstärkte das den pathogenen Phänotyp verglichen mit der jeweils singulären Überexpression erheblich. Solch ein synergistischer onkogener Effekt lässt sich auch durch Hemmung des Tumorsupressors p53 in Kombination mit erhöhtem CK2-Niveau feststellen. Mäuse, die heterozygot für p53 sind und eine gesteigerte CK2α-Expression besitzen, entwickeln T-Zell Lymphome im Durchschnitt doppelt so schnell wie p53-heterozygote Mäuse (Landesman-Bollag *et al.*, 1998). Andere transgene Mäuse mit einer verstärkten CK2α-Expression in den Brustdrüsen entwickelten in diesem Gewebe rasch Hyper- und Neoplasien. In den transgenen Mausmodellen mit ausschließlich erhöhter CK2α-Expression, war eine Latenz in der Tumorentwicklung festzustellen (Landesman-Bollag *et al.*, 2001a). Dies deutet darauf hin, dass die erhöhte CK2-Aktivität für die Onkogenese alleine nicht hinreichend ist. Es wird vielmehr diskutiert, dass die Transformation dieser Mäuselymphozyten durch einen mehrstufigen Prozess ausgelöst wurde (Xu *et al.*, 1999a; Landesman-Bollag *et al.*, 2001b). Die beobachtete schnellere Onkogenese nach zusätzlich gesteigerter Onkogen- bzw. verminderter Tumorsupressorgen-Expression unterstützt diese Hypothese. In Kombination mit den zuvor beschrieben Erkenntnissen kann man annehmen, dass eine übermäßige CK2-Aktivität ein zelluläres Milieu schafft, welches dem unkontrollierten Wachstum Vorschub leistet und der Apoptose entgegenwirkt. Solche Zellen sind der meisten natürlichen Abwehrmechanismen gegen sekundäre, kanzerogene Ereignisse beraubt und erliegen schneller einer neoplastischen Transformation. Strenggenommen stellt die CK2 selbst demnach kein Onkoprotein dar (Trembley *et al.*, 2009). Falls nicht schon zu Beginn der Onkogenese die Abhängigkeit von erhöhter CK2-Aktivität besteht, scheinen viele Tumorzellen diese mit fortschreitender Progression zu entwickeln (Ruzzene und Pinna, 2010).

In verschiedenen Studien konnte durch eine Hemmung der CK2 das Wachstum von Tumorzellen begrenzt oder sogar eine Remission des Tumorphänotyps erreicht werden. Auswirkungen einer Unterdrückung der CK2-Biosynthese und einer daraus resultierenden geringen CK2-Menge im Tumorgewebe wurden beispielsweise im murinen Xenograft-Modell des Prostatakarzinoms untersucht (Slaton *et al.*, 2004). Dort wurde die CK2-Expression *in vivo* durch intratumorale Injektion von antisense RNA gegen CK2α reduziert. Eine einmalige Administration von 5 µg antisense RNA reduzierte die Tumorgröße innerhalb von 7 Tagen auf 30-40 %. Eine Dosiserhöhung auf 20 µg führte nach 7 Tagen zu einer völligen Rückbildung des Tumors. Diese beeindruckenden Ergebnisse bestätigten die Abhängigkeit des Tumors von der CK2α. Sie klärten aber nicht, ob tatsächlich deren katalytische Aktivität oder nicht-katalytische Wechselwirkungen für die tumorfördernde Wirkung verantwortlich waren. So wertvoll antisense-Oligonukleotide in der Grundlagenforschung sind, so begrenzt ist noch die Möglichkeit, sie zu therapeutischen Zwecken bei Krebserkrankungen einsetzen zu können. Zur klinischen Anwendung werden nach wie vor bevorzugt niedermolekulare Inhibitoren entwickelt. In einigen Experimenten wurde dazu der Effekt zellpermeabler, niedermolekularer CK2-Inhibitoren auf kultivierte Krebszelllinien untersucht. So induzierten DRB (Farah *et al.*, 2003), Emodin (Olsen *et al.*, 2007), TBB (Sarno *et al.*, 2001), TBI (Zien *et al.*, 2005), DMAT (Pagano *et al.*, 2004), IQA (Sarno *et al.*, 2003) und TBCA (Pagano *et al.*, 2007) die Apoptose und wirkten zytotoxisch. Die Effekte dieser ATP-kompetitiven Inhibitoren ergeben sich höchstwahrscheinlich aus der Hemmung der in den Krebszellen erhöhten CK2-Aktivität. Für einige Inhibitoren wie Emodin, TBCA und TBB wurde darüber hinaus eine Hemmung der Angiogenese nachgewiesen (Kramerov *et al.*, 2008). Eine Senkung der CK2-Aktivität wirkt demnach einigen Hauptmerkmalen der Onkogenese entgegen.

Alle bisherigen Erkenntnisse belegen die Abhängigkeit der Krebserkrankungen von erhöhter CK2-Aktivität. Insgesamt untermauern die aufgeführten Ergebnisse die Annahme, dass in erster Linie die katalytische Aktivität für die pathologischen Effekte des erhöhten CK2-Niveaus in Krebserkrankungen verantwortlich ist. Darüber hinaus konnte gezeigt werden, dass eine Hemmung der CK2-Aktivität dem Tumor-Phänotyp entgegenwirkt. Die CK2 ist demnach ein attraktives Zielmolekül für die Entwicklung von Wirkstoffen zur therapeutischen Intervention bei Krebserkrankungen.

Einer hohen Aktivität der Proteinkinasen kann auf Proteinebene durch niedermolekulare Wirkstoffe begegnet werden. Die ersten Inhibitoren von Proteinkinasen wurden vor über 25 Jahren identifiziert und zunächst hauptsächlich zur Aufklärung der physiologischen Funktion dieser Enzymklasse eingesetzt (Hidaka *et al.*, 1984). Der überwältigende Erfolg von Imatinib beflügelte die Hoffnung auf einen breiten klinischen Einsatz dieser Wirkstoffgruppe gegen onkologische Erkrankungen (Druker, 2009). Die Entwicklung des Proteinkinase-Inhibitors Imatinib zielte darauf ab, das Onkoprotein Bcr-Abl zu hemmen. Seit diesen bahnbrechenden Arbeiten hat die Erforschung und Entwicklung von Proteinkinase-Inhibitoren stark an Bedeutung gewonnen. Schätzungen zufolge beschäftigen sich Zurzeit über 30 % der industriellen Programme zur Wirkstoffentwicklung mit Inhibitoren von Proteinkinasen (Cohen, 2002b; Eglen und Reisine, 2009). Diese Wirkstoffklasse ist damit zu einem der bedeutendsten pharmazeutischen Forschungsschwerpunkte geworden.

Um die Potenz eines Inhibitors bestimmen zu können, ist ein verlässlicher und aussagekräftiger Aktivitätstest für das Zielenzym unerlässlich. Die z. Zt. meist genutzte Methode, um die Aktivität von Proteinkinasen zu bestimmen, ist ein radiometrischer Test (Hastie *et al.*, 2006). In publizierten Untersuchungen zur CK2-Aktivität wird fast ausschließlich ein radiometrischer Filterbindungstest als Standardmethode eingesetzt (Bretner *et al.*, 2008; Schneider *et al.*, 2009). Dieser basiert auf der Quantifizierung des Transfers [³²P]-markierten γ-Phosphats von ATP auf eine Serylseitenkette eines Peptid- oder Protein-Substrats durch CK2. Ein Peptid, das für viele CK2-Aktivitätstests eingesetzt wird, hat die AS-Sequenz RRRDDDSDDD und kann über die N-terminalen Arginine an ein Kationenaustauscher-Filterpapier gebunden werden. Nicht umgesetztes γ-[³²P]-ATP bindet nicht und kann abgewaschen werden. Die verbleibende Radioaktivität kann quantifiziert werden und ist proportional zur CK2-Aktivtät (Hastie *et al.*, 2006). Die radiometrische Detektion des Phosphotransfers ist ein Grundprinzip, das eine Aktivitätsbestimmung nahezu aller Proteinkinasen erlaubt. Zu diesem Zweck muss ein CK2-Substrat verwendet werden, das man zur Analyse z. B. über ionische Wechselwirkungen immobilisieren kann. Weiterhin wird in kurzen Intervallen neues γ-[³²P]-ATP benötigt, da die Halbwertszeit des ³²P-Isoptops gering ist. Somit fällt ständig radioaktiver Abfall an. Ein radiometrischer Kinasetest ist daher nur mit großem Aufwand automatisierbar und ist besonders bei geringem Probendurchsatz recht aufwendig.

Es gibt mehrere alternative Methoden, die Aktivität von Proteinkinasen nicht-radiometrisch zu bestimmen (Jia *et al.*, 2008; Ma *et al.*, 2008). Zur effektiven Messung der CK2-Aktivität wurde bislang allerdings nur ein einziges nicht-radiometrisches Testverfahren in der Literatur beschrieben (Hung *et al.*, 2009). Dieses auch kommerziell erhältliche Reagenzienset ("CK2 Kinase Assay/Inhibitor Screening Kit", CycLex, Nagano, Japan) nutzt einen Peroxidasegekoppelten anti-phospho-p53-Antikörper, der an Ser-46 in p53 bindet, sofern diese Seitenkette durch CK2 phosphoryliert wurde. Dieser Antikörper dient als Reporter in einem homogenen ELISA-Ansatz. Er entwickelt nach Inkubation mit dem chromogenen Substrat Tetramethylbenzidin ein farbiges Produkt, das spektroskopisch detektiert werden kann. Durch Absorptionsmessung kann die Menge an umgesetztem CK2-Substrat quantifiziert werden. Vergleichende Untersuchungen der CK2-Aktivität mit diesem Testverfahren stehen allerdings aus. Bislang sind damit nur wenige semi-quantitative Bestimmungen beschrieben worden (Hung *et al.*, 2009). Daneben existieren indirekte Tests, z. B. eine luminometrische Detektion der Abnahme des Cosubstrats ATP während der Kinasereaktion mittels Luziferase ("Kinase-Glo assay", Promega, Mannheim).

Für die Inhibitionsbestimmungen, die in dieser Arbeit durchgeführt werden sollten, wurde ein Test benötigt, der mehrere Voraussetzungen erfüllt. Er sollte auf Radioisotope verzichten sowie möglichst einfach durchzuführen und dabei reproduzierbar und robust sein. Darüber hinaus sollte er sich zur Reihenuntersuchung von niedermolekularen Verbindungen eignen und im Idealfall die Bestimmung von IC₅₀ Werten ermöglichen. Die Ergebnisse sollten nicht nur untereinander vergleichbar sein, sondern auch einen Vergleich zu Literaturdaten ermöglichen. Keiner der veröffentlichten CK2-Inhibitionstests genügte diesen Anforderungen.

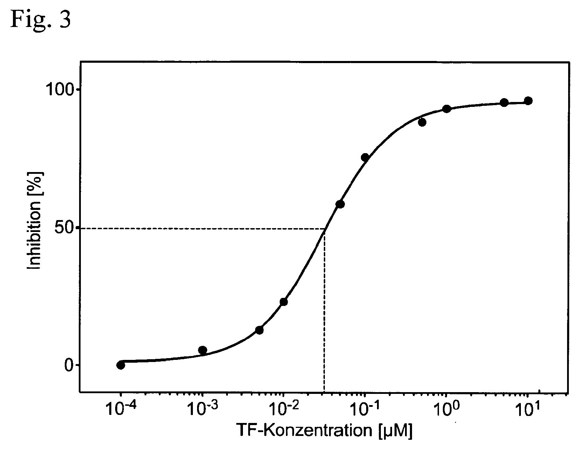

Bei den bisher potentesten (IC₅₀ < 1 µM) und am besten untersuchten Inhibitoren der CK2 handelt es sich um niedermolekulare Substanzen, welche die Cosubstratbindestelle des aktiven Zentrums adressieren (Figur 3). Aktuelle Übersichten zu bisher bekannte Inhibitoren der CK2 bieten Publikationen von Bortolato *et al.* (2008), Battistutta (2009), Cozza *et al.* (2010) sowie Prudent und Cochet (2010). Einige wichtige Verbindungen mit verschiedenen Grundgerüsten werden im Folgenden exemplarisch vorgestellt.

Viele der heute bekannten CK2-Inhibitoren leiten sich vom Benzimidazol-Grundgerüst des DRB (5,6-Dichloro-1-β-D-ribofuranosylbenzimidazol) ab. DRB ist ein Adenosin-Analogon und wurde als einer der ersten CK2-Inhibitoren identifiziert (Zandomeni *et al.*, 1986). Sein hoher IC₅₀ Wert von 13 µM und seine mäßige Selektivität unter verschiedenen Proteinkinasen führten jedoch zu seiner heutigen geringen Relevanz als CK2-Inhibitor. Strukturvariationen von DRB führten hingegen zu potenteren Verbindungen. Dabei zeigte sich, dass der Zuckerrest für die Hemmung keine Rolle spielt, der Austausch der zwei Chlor- gegen vier Brom-Substituenten am Benzimidazol-Grundkörper die inhibitorische Potenz jedoch deutlich steigert (Meggio *et al.*, 1990). Dies führte zur Entwicklung der zwei halogenierten Benzimidazol-Derivate TBI (4,5,6,7-Tetrabrombenzimidazol) und TBB (4,5,6,7-Tetrabrombenzotriazol, Figur 3), welche beide einen IC₅₀ Wert um 1 µM besitzen. Wichtiger als die Potenz waren allerdings die gute Zellpermeabilität und die relativ hohe Selektivität, die v. a. TBB in Untersuchungen mit 33 anderen Proteinkinasen zeigte (Sarno *et al.*, 2001). Diese Eigenschaften machten TBB in der Erforschung biologischer Funktionen von CK2 zum Inhibitor erster Wahl. Durch weitere Variation des TBI-Grundgerüstes konnte zuletzt der Inhibitor DMAT (2-Dimethylamino-4, 5, 6, 7-tetrabrombenzimidazol) identifiziert werden, dessen IC₅₀ Wert im Vergleich zu TBB um knapp eine Zehnerpotenz auf 0,14 µM gesenkt werden konnte. Er stellt einen der potentesten Vertreter dieser halogenierten Benzimidazol-Derivate dar (Pagano *et al.*, 2004). In einer breit angelegten Selektivitätsuntersuchung fand man allerdings, dass DMAT auch andere Proteinkinasen, z. B. PIM 1 ("proto-oncogene serine/ threonine-protein kinase 1") und DYRK1a ("dual-specificity tyrosine-phosphorylated and -regulated kinase 1a"), mit ähnlicher Potenz hemmt (Sarno *et al.*, 2003; Pagano *et al.*, 2008), was seine Bedeutung als selektiver CK2-Inhibitor schmälerte. Aus diesem Grund wird in den letzten Jahren in erster Linie an einer Selektivitätssteigerung der Benzimidazol-Derivate gearbeitet (Pagano *et al.*, 2008).

Mehrere polyphenolische CK2-Inhibitoren konnten aus Pflanzenextrakten isoliert werden. Eine Reihe natürlicher Flavonoide, wie Apigenin (aus *Apium graveolens,* Figur 3), Luteolin oder Quercetin zeigen zwar IC₅₀ Werte um 1 µM (Li *et al.*, 2009), hemmen aber zugleich mehrere andere Proteinkinasen, teilweise sogar deutlich stärker als die CK2. Deutlich selektiver ist Hämatein, das einen IC₅₀ Wert von 0,55 µM besitzt (Hung *et al.*, 2009). Auch einige Anthrachinon-Derivate sind CK2-Inhibitoren. Ein potenter Vertreter dieser Gruppe ist Emodin aus *Rheum palmatum* (Figur 3), das bei mäßiger Selektivität für CK2 einen IC₅₀ Wert um 1 µM zeigt (Yim *et al.*, 1999). Sowohl seine Potenz als auch seine Selektivität konnte durch Modifikationen verbessert werden. So hat MNX (1,8-Dihydro-4-nitroxanthen-9-on) beispielsweise einen IC₅₀ Wert von 0,4 µM und Chinalizarin (1, 2, 5, 8-Tetrahydroxyanthrachinon) einen IC₅₀ Wert von 0,11 µM (Cozza *et al.*, 2009). Durch Modifikationen des Grundgerüsts zu Cumarin-Derivaten konnten weitere Inhibitoren entwickelt werden, die einige Nachteile der Anthrachinon-Derivate beseitigten. Ein potenter Vertreter dieser Cumarin-Derivate ist DBC (3,8-Dibrom-7-hydroxy-4-methyl-chromen) mit einem IC₅₀ Wert von 0,1 µM. Die Ellagsäure (Figur 3) wurde als bisher potentester polyphenolischer CK2-Inhibitor aus dem pflanzlichen Sekundärstoffwechsel identifiziert. Sie besitzt einen IC₅₀ Wert von 0,04 µM und zeigte im Vergleich zu 12 anderen Proteinkinasen eine selektive Hemmung der CK2 (Cozza *et al.*, 2006).

In den letzten Jahren haben technische Fortschritte wie computergestützte Methoden oder "library screening" dazu geführt, dass CK2-Inhibitoren mit bislang unbekannten Grundstrukturen identifiziert wurden. Beispielsweise wurde die synthetische IQA (5-Oxo-5,6-dihydroindolo[1,2-*a*]chinazolin-7-yl)essigsäure, Figur 3) wie die bereits erwähnte Ellagsäure durch computergestützte "virtual screening"-Methoden als CK2-Inhibitor identifiziert (Sarno *et al.*, 2003). Das Indolochinazolin-Derivat IQA unterscheidet sich strukturell von allen anderen beschriebenen Inhibitoren, hat einen IC₅₀ Wert von 0,39 µM und zeigt selektive CK2-Hemmung unter 44 Proteinkinasen (Sarno *et al.*, 2003). Es ist damit zwar weniger potent als andere Inhibitoren, zeigt aber eine größere Selektivität und lässt weitere Strukturmodifikationen zu. Weiterhin wurde so auch TBCA (Tetrabromzimtsäure) identifiziert, die einen IC₅₀ Wert von 0,11 µM besitzt und unter 28 gestesteten Proteinkinasen selektiv für CK2 war (Pagano *et al.,* 2007). Kürzlich wurden planare, teilweise makrozyklische Pyrazolo [1,5-*a*][1,3,5]triazine beschrieben, die in initialen Zellkulturtests starke inhibitorische Aktivität gegenüber CK2 zeigten (Nie *et al.,* 2008) und z. Zt. weiter untersucht werden. Ebenfalls neueren Datums sind die Hemmdaten substituierter Pyrazin-Grundstrukturen. Derivate dieser Substanzklasse zeigten in ersten Hemmversuchen mit der CK2α IC₅₀ Werte im nanomolaren Bereich (Suzuki *et al.,* 2008).

Die Cosubstratkavität ist bislang die Zielstruktur, an der die potentesten Inhibitoren der CK2 binden. Die ATP-Konzentration in der Zelle liegt zwischen 1 mM und 10 mM (Cohen, 1999). Mögliche ATP-kompetitive Inhibitoren müssen eine hohe Affinität aufweisen, um ATP unter diesen Umständen aus seiner Bindetasche zu verdrängen. Zudem ist genau diese Struktur innerhalb verschiedener Proteinkinasen am wenigsten variiert. Deshalb ist es schwierig, selektive ATP-kompetitive Inhibitoren für die CK2 zu entwickeln. Eine selektive Adressierung des Zielmoleküls ist allerdings eine Voraussetzung für eine potentielle klinische Anwendung. Darüber hinaus sollte ein Arzneistoffkandidat klassische Anforderungen der Medizinischen Chemie, wie Lipinskis "rule of five" erfüllen (Lipinski *et al.,* 2001). Er muss wasserlöslich, zellpermeabel und unter physiologischen Bedingungen stabil sein, um eine ausreichende Bioverfügbarkeit zu gewährleisten.

Ein großer Nachteil vieler Anthrachinon-Derivate wie Emodin ist deren potentielle Interkalation in DNA (Wang *et al.,* 2006). Diese Eigenschaft macht die Substanzgruppe für CK2-spezifische Untersuchungen in zellulären Systemen und zur gezielten therapeutischen CK2-Hemmung unbrauchbar. TBB hingegen ist zwar gut für *in* vitro-Experimente geeignet, aber aufgrund seiner relativ schlechten Wasserlöslichkeit ist dieser CK2-Inhibitor nicht für den klinischen Einsatz geeignet (Battistutta *et al.,* 2000). Auch IQA ist zwar potent und sehr selektiv für die CK2, ist aber im wässrigen Milieu instabil, da sein Lactamring anfällig für eine langsame Hydrolyse ist (Sarno *et al.,* 2003; Sarno *et al.,* 2005). Wie an diesen Beispielen gezeigt, erfüllen bislang nur sehr wenige der veröffentlichten ATP-kompetitiven CK2-Inhibitoren grundlegende Anforderungen an einen Arzneistoffkandidat. Das ist auch ein Grund für die Entwicklung von Inhibitoren mit alternativen Wirkmechanismen, die zunehmend an Bedeutung gewinnen. Für DRB fiel durch Cokristallisationen mit CK2 eher zufällig auf, dass diese Verbindung neben einer Affinität zur Cosubstratkavität auch eine Affinität zu einer hydrophilen Bindetasche an der Kontaktfläche zur β-Untereinheit aufwies. Diese Bindetasche könnte als allosterisches Ziel neuer selektiver Inhibitoren eine Tetramerisierung der CK2 verhindern und somit ihre Regulation beeinflussen. Darüber hinaus wurden in den letzten drei Jahren diverse Substanzen gefunden, die effektiv eine Bindung von CK2α und CK2β verhindern (Prudent *et al.,* 2010). So konnten von CK2β zyklische 11-mer Peptide abgeleitet werden, die in niedriger mikromolarer Konzentration eine Bildung des CK2α-CK2β Komplexes blockieren (Laudet *et al.,* 2007). Basierend auf diesen Ergebnissen ist es derselben Gruppe gelungen, niedermolekulare Substanzen zu entwickeln, die diese allosterische Bindetasche adressieren. Dabei handelt es sich um Podophyllotoxin-indolo-Derivate, die als nicht-kompetitive Inhibitoren IC₅₀ Werte bis zu 20 µM zeigen (Laudet *et al.,* 2008). Auch anorganische Substanzen können als allosterische Inhibitoren wirken. Beispielsweise wurden für einige Polyoxometallate IC₅₀ Werte im einstelligen nanomolaren Bereich sowohl für CK2α als auch für das Holoenzym festgestellt (Prudent *et al.,* 2008). Neben dem CK2α-CK2β Kontakt bietet die acidophile Substratbindestelle der CK2α eine noch wenig adressierte, attraktive Struktur zur Entwicklung selektiver Inhibitoren. In einer selten zitierten Studie einer kubanischen Arbeitsgruppe wurde von einem zyklischen Peptid mit Affinität zur CK2α berichtet (AS-Sequenz CWMSPRHLGTC) (Perea *et al.,* 2004). Die Autoren entwickelten das Fusionspeptid CIGB-300, das aus dieser Sequenz und einem zellpermeablen Peptid besteht, welches von dem HIV-Tat Protein abgeleitet ist. *In vivo* Studien zeigten, dass CIGB-300 pro-apoptotisches Potential besitzt und eine Wachstumshemmung von Tumorgewebe bewirkt (Perea *et al.,* 2008). In dieser Studie blieb jedoch offen, ob die pro-apoptotische Aktivität von CIGB-300 tatsächlich auf eine Hemmung der CK2 zurückgeführt werden kann. Neuere Untersuchungen berichten nicht mehr von einer generellen Inhibition der CK2 durch CIGB-300 sondern von einer Interaktion zwischen CIGB-300 und Nucleophosmin, einem Substrat der CK2 (Perera *et al.,* 2009).

Es ist anzunehmen, dass neben den publizierten Ergebnissen der Wirkstoffforschung mehrere industrielle Programme zur Entwicklung von CK2-Inhibitoren betrieben werden. Beispielsweise wurde erst kürzlich bekannt, dass mit dem Wirkstoff CX-4945 (Figur 3) der erste niedermolekularer CK2-Inhibitor in einer klinischen Phase-I-Studie als orales Krebstherapeutikum untersucht wird (Chua *et al.,* 2008; Pinna und Allende, 2009). In der präklinischen Untersuchung zeigte CX-4945 eine potente und selektive Hemmung der CK2, sowie gute anti-tumorale Eigenschaften in Zelllinien und Xenograft-Krebsmodellen (Dancey, 2009).

Wirkstoffe, welche die Aktivität der CK2 modulieren, stellen wertvolle Werkzeuge dar, mit deren Hilfe (patho)physiologische Funktionen dieses Enzyms aufgeklärt werden können. Neben der Variation vorhandener CK2-Inhibitoren ist die Erkundung neuer Leitstrukturen ein wichtiger Beitrag zur Entwicklung potenter und selektiver Wirkstoffe, die sich auch für die klinische Anwendung eignen. Erste Erfolge bestärken die Hoffnung, dass CK2-Inhibitoren identifiziert werden können, die als gezielte Arzneistoffe in der Tumortherapie eingesetzt werden können.

Kuckländer et al. "Zur Umsetzung von 2-(Aminomethylen)cyclohexanon-Derivaten mit Dichlorchinonen", Chemische Berichte, Bd. 116, 1983, Seite 152-153, beschreibt allgemein die Umsetzung von Dichlor-p-benzochinonen mit 2-[(Arylamino)methylen]cyclohexanon-Derivaten. Durch Umsetzung von Dichlor-p-benzochinonen 1 mit 2-[(Arylamino)-methylen]cyclohexanon-Derivaten werden Dibenzofuran-Abkömmlinge erhalten. Ihre Struktur wird spektroskopisch belegt und durch Oxidation sowie anschließende Hydrolyse chemisch bewiesen.

Das zuvor Ausgeführte berücksichtigend ist es daher die Aufgabe der vorliegenden Erfindung, neue Verbindungen zur selektiven Hemmung von Proteinkinasen sowie ein Medikament und/oder Arzneimittel welches solche neuen Verbindungen aufweist, anzugeben.

Gelöst wird diese Aufgabe hinsichtlich der Verbindung und des Medikaments bzw. Arzneimittels durch eine Verbindung gemäß Anspruch 1.

Überraschender Weise hat sich gezeigt, dass Dibenzofuranderivate gemäß allgemeinen Formel I in der Lage sind, Kinasen, insbesondere Serin-/Threoninkinasen selektiv zu hemmen und so eine gezielte Apoptose neoplastischen Gewebes einzuleiten.

In einer Ausgestaltung der Erfindung ist R1 eine parasubstituierte Arylgruppe ist, R2 und R3 unabhängig voneinander F, Cl oder H sind, R4 H oder CH₃CO ist, und R5 H.

In einer weiteren Ausgestaltung der erfindungsgemäßen pharmazeutischen Zubereitung ist R1 = 4-CH₃OC₆H₄, 4-CH₃C₆H₄ oder 4-FC₆H₆.

In einer weiteren, bevorzugten Ausgestaltung ist R1 = 4-CH₃C₆H₄; R2 und R3 = Cl; und R4 und R5 = H.

In einer weiteren, bevorzugten Ausgestaltung ist R1 = 4-CH₃OC₆H₄; R3 und R5 = Cl; R2 und R4=H.

Die erfindungsgemäße pharmazeutische Zubereitung kann weitere pharmazeutisch gebräuchliche Zusätze und Hilfsstoffe enthalten. Darüber hinaus kann diese wenigstens eine weitere antineoplastisch wirkende Verbindung aufweisen.

Alkyl- oder Acylgruppe mit 1 bis 16 C-Atomen, vorzugsweise (CₙH₂ₙ₊₁)-CO mit n=1, 2, 3, 4, 5, 6, 7, 8 ist, als Diagnostikum zur Untersuchung der Rolle von Proteinkinasen, insbesondere Serin-/Threoninkinasen, insbesondere der Proteinkinase CK2 bei zellulären Prozessen, der Pathogenese von Erkrankungen, der Ontogenese und/oder sonstigen entwicklungsbiologischen Phänomenen oder Zusammenhängen gelöst.

Darüber hinaus hat sich gezeigt, dass Verbindungen der allgemeinen Formel I 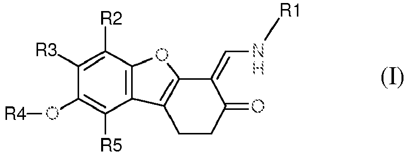 wobei R1 eine substituierte oder unsubstituierte Arylgruppe oder eine substituierte oder unsubstituierte Alkylgruppe mit 1 bis 16 C-Atomen, vorzugsweise eine Halogen, Alkoxy und/oder Alkyl substituierte Arylgruppe ist; R2, R3 und R5 unabhängig voneinander F, Cl, Br, I, H, eine Hydroxylgruppe oder Alkoxygruppe mit 1 bis 4 C-Atomen sind; und R4 H, eine Alkyl- oder Acylgruppe mit 1 bis 16 C-Atomen, vorzugsweise (CₙH₂ₙ₊₁)-CO mit n=1, 2, 3, 4, 5, 6, 7, 8 ist, antioxidative Eigenschaften zeigen und so als antioxidativer Wirkstoff zur Herstellung von Pharmazeutika, insbesondere zur präventiven Anwendung, verwendet werden können.

Darüber hinaus hat sich gezeigt, dass Verbindungen der allgemeinen Formel II 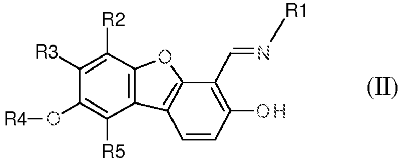 wobei R1 eine substituierte oder unsubstituierte Arylgruppe oder eine substituierte oder unsubstituierte Alkylgruppe mit 1 bis 16 C-Atomen, vorzugsweise eine Halogen, Alkoxy und/oder Alkyl substituierte Arylgruppe ist; R2, R3 und R5 unabhängig voneinander F, Cl, Br, I, H, eine Hydroxylgruppe oder Alkoxygruppe mit 1 bis 4 C-Atomen sind; und R4 H, eine Alkyl- oder Acylgruppe mit 1 bis 16 C-Atomen, vorzugsweise (CₙH₂ₙ₊₁)-CO mit n=1, 2, 3, 4, 5, 6, 7, 8 ist, ebenfalls als Wirksubstanzen in pharmazeutischen Zubereitungen eingesetzt werden können. Hierbei hat sich ebenfalls in überraschender Weise gezeigt, dass Verbindungen gemäß allgemeinen Formel II in der Lage sind, Kinasen, insbesondere Serin-/Threoninkinasen selektiv zu hemmen und so eine gezielte Apoptose neoplastischen Gewebes einzuleiten.

In einer bevorzugten Ausgestaltung ist dabei in Formel II R1 = 4-CH₃OC₆H₄; R3 und R5 = Cl; R2 und R4 = H.

Die Erfindung ist nachfolgend anhand von Beispielen näher erläutert.
- Fig. 1: zeigt die Laterale Wirkung von CK2 auf (A) Wnt-, (B) Akt-, und (C) NF-κB-Signaltransduktion;
- Fig. 2: zeigt Vertreter ATP-kompetitiver Inhibitoren mit unterschiedlichen Grundgerüsten, die bereits in mikromolarer Konzentration eine Wirkung auf die Aktivität der CK2 zeigen;
- Fig. 3: zeigt die Bestimmung des IC₅₀ Wertes von TF;
- Fig. 4: zeigt den Effekt von TF (A) und TBB (B) auf die Viabilität von LNCaP-Zellen;
- Fig. 5: zeigt den Effekt von TF und TBB auf die PARP-Spaltung in LNCaP-Zellen;
- Fig. 6: zeigt die CK2-Aktivität in Lysaten von LNCaP-Zellen, die zuvor mit TF (A) oder TBB (B) inkubiert wurden;
- Fig. 7: zeigt das Selektivitätsprofil von TF mit einer Auswahl von 63 humanen Proteinkinasen;
- Fig. 8: zeigt das Syntheseschema zur Synthese von Verbindungen der allgemeinen Formel I.
- Fig. 9: zeigt die Untersuchung des Inhibitionsmodus der Verbindung TF;

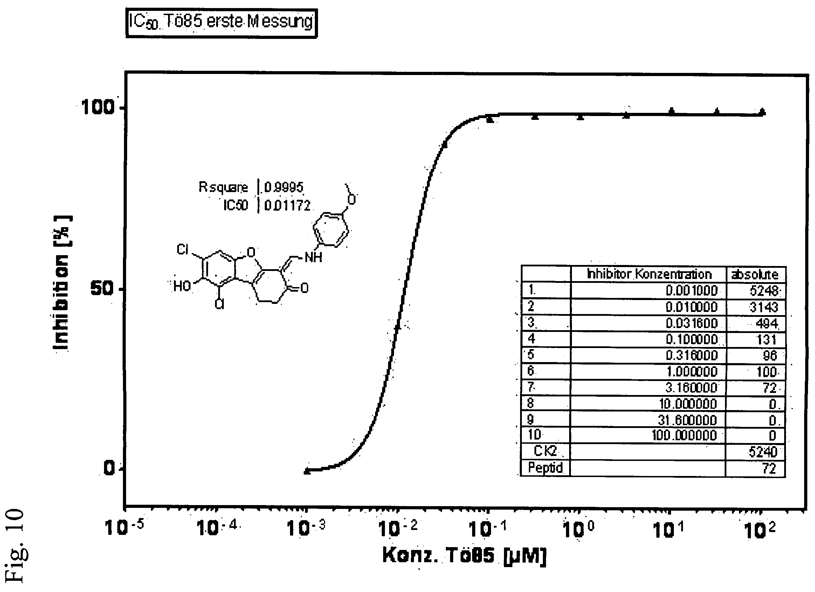

- Fig. 10: zeigt eine erste Bestimmung des IC₅₀ Wertes der Verbindung Tö85, bei welcher die allgemeine Formel I Substitutionsmuster R1 = 4-CH₃OC₆H₄; R3 und R5 = Cl; R2 und R4 = H aufweist;

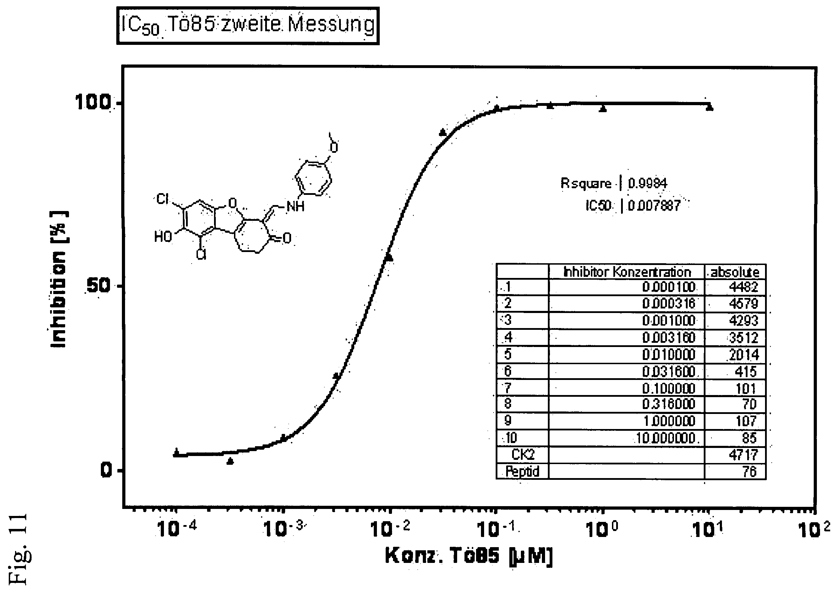

- Fig. 11: zeigt eine zweite Bestimmung des IC₅₀ Wertes von Tö85;

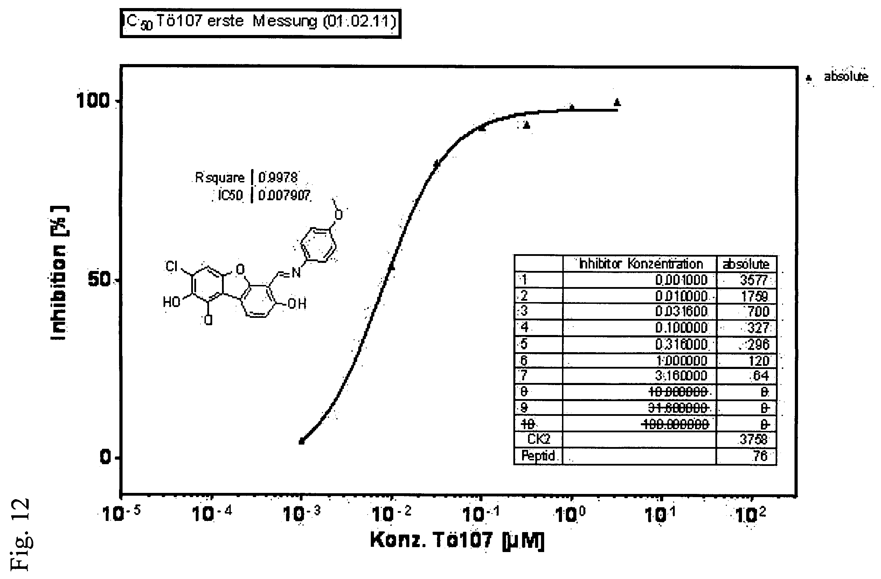

- Fig. 12: zeigt eine erste Bestimmung des IC₅₀ Wertes der Verbindung Tö107, bei welcher die allgemeine Formel II Substitutionsmuster R1 = 4-CH₃OC₆H₄; R3 und R5 = Cl; R2 und R4 = H aufweist;

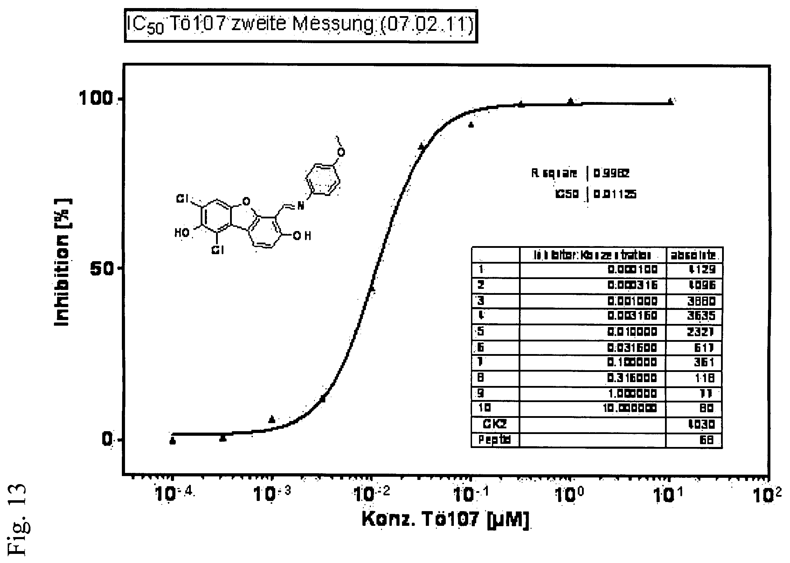

- Fig. 13: zeigt eine zweite Bestimmung des IC₅₀ Wertes von Tö107;

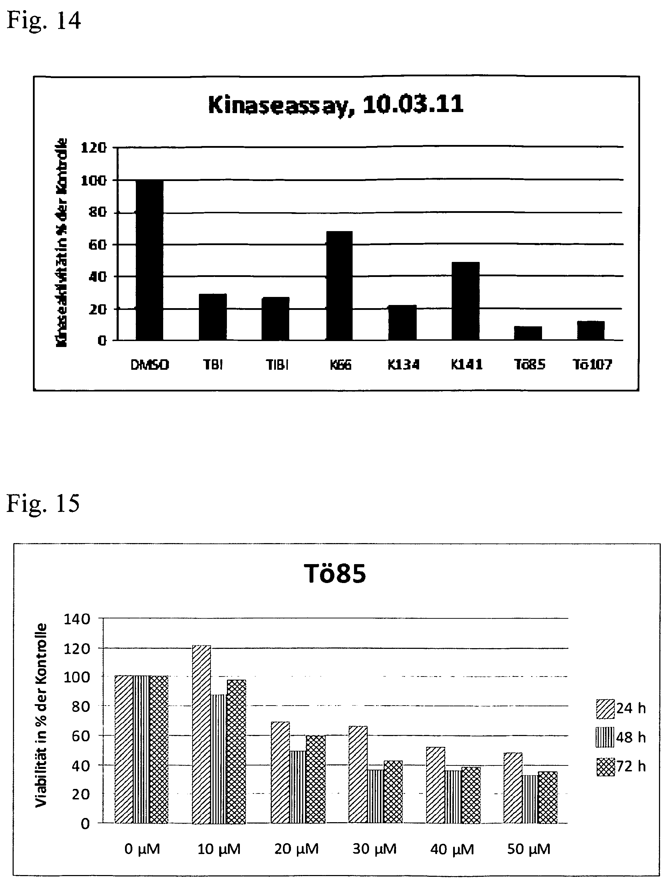

- Fig. 14: zeigt die Hemmung der Proteinkinase CK2 in Zellen der Prostatakrebszelllinie LNCaP durch Tö85 und Tö107 im Vergleich zu den Verbindungen DMSO, TBI, TIBI, K66, K134 und K131;
- Fig. 15: zeigt die Konzentrationsabhängige Reduktion der Viabilität von Zellen der Prostatakrebszelllinie LNCaP durch Tö85;

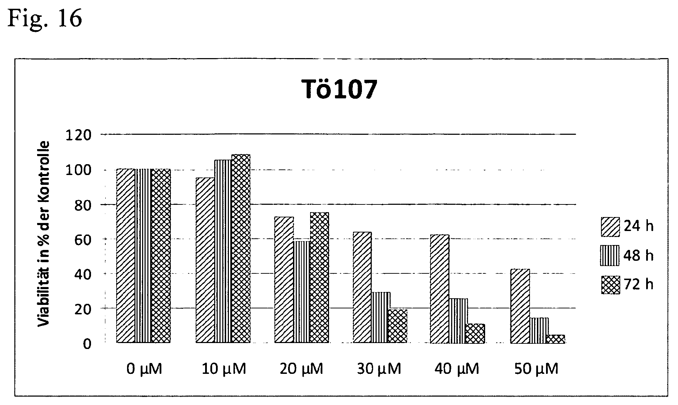

- Fig. 16: zeigt die Konzentrationsabhängige Reduktion der Viabilität von Zellen der Prostatakrebszelllinie LNCaP durch Tö107.

In Fig. 1 ist die laterale Wirkung von CK2 auf (A) Wnt-, (B) Akt-, und (C) NF-κB-Signaltransduktion gezeigt. Negative Effekte (-) sind durch punktköpfige Pfeile gekennzeichnet und basieren auf Inhibition bzw. auf verstärktem Abbau. Positive Effekte (+) sind durch normale Pfeile gekennzeichnet und basieren auf verstärkter Stabilität und/oder Aktivität (verändert nach Ruzzene und Pinna (2010)).

In Fig. 2 sind Vertreter ATP-kompetitiver Inhibitoren mit unterschiedlichen Grundgerüsten aus dem Stand der Technik gezeigt, die bereits in mikromolarer Konzentration eine Wirkung auf die Aktivität der CK2 zeigen.

Fig. 3 zeigt die Bestimmung des IC₅₀ Wertes von TF. Dazu wurde der kapillarelektrophoretische CK2-Inhibitionstest mit E-Peptid als Substrat eingesetzt. Die Inhibition von CK2 wurde nach zehnminütiger Präinkubation mit TF gemessen. Dabei wurden zehn Konzentrationen zwischen 0,0001 µM und 10 µM untersucht.

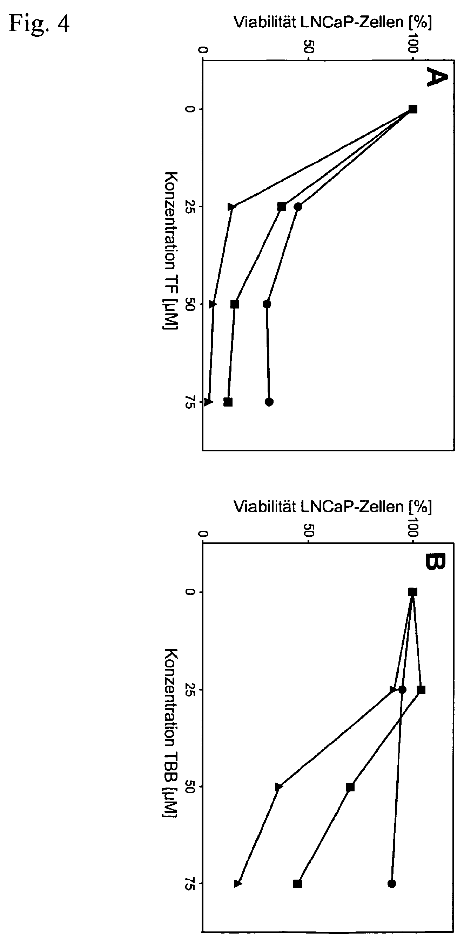

Fig. 4 zeigt die Effekt von TF (A) und TBB (B) auf die Viabilität von LNCaP-Zellen. Die Viabilität wurde mit Hilfe des MTT-Tests bestimmt. In jedem Diagramm ist der konzentrationsabhängige Effekt nach einer Inkubationsdauer von 24 h (●), 48 h ( ) und 72 h (▲) aufgetragen.

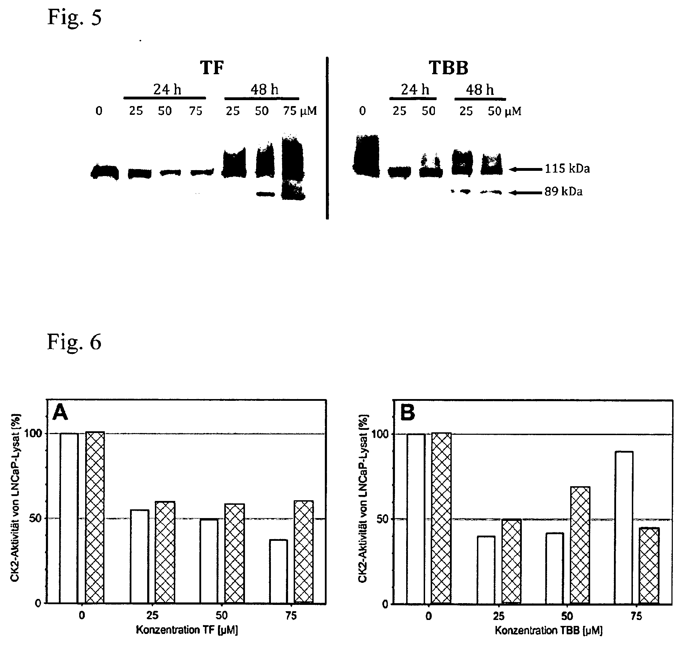

Fig. 5 zeigt den Effekt von TF und TBB auf die PARP-Spaltung in LNCaP-Zellen. Die Zellen wurden 24 h oder 48 h mit 25 µM, 50 µM und 75 µM TF oder mit 25 µM, 50 µM TBB inkubiert. Als Kontrolle dienten Zellen, die nur mit DMSO behandelt wurden (0 µM). Nach SDS-PAGE und Western Blotting wurden vollständiges PARP (115 kDa) und das durch Caspasespaltung entstehende 89 kDa große Fragment mit Hilfe eines Anti-PARP Antikörper markiert.

Fig. 6 zeigt die CK2-Aktivität in Lysaten von LNCaP-Zellen, die zuvor mit TF (A) oder TBB (B) inkubiert wurden. Die CK2-Aktivität wurde mit dem radiometrischen Testverfahren ermittelt. Die relativen Aktivitäten der Proben wurden jeweils auf unbehandelte Zellen bezogen. Weiße Balken geben die CK2-Aktivität nach 24 h, graue Balken die CK2-Aktivität nach 48 h Inkubation mit dem jeweiligen Inhibitor an. Für mit TF behandelte Proben (A) wurden Doppelbestimmungen durchgeführt (angegeben ist dort jeweils der MW).

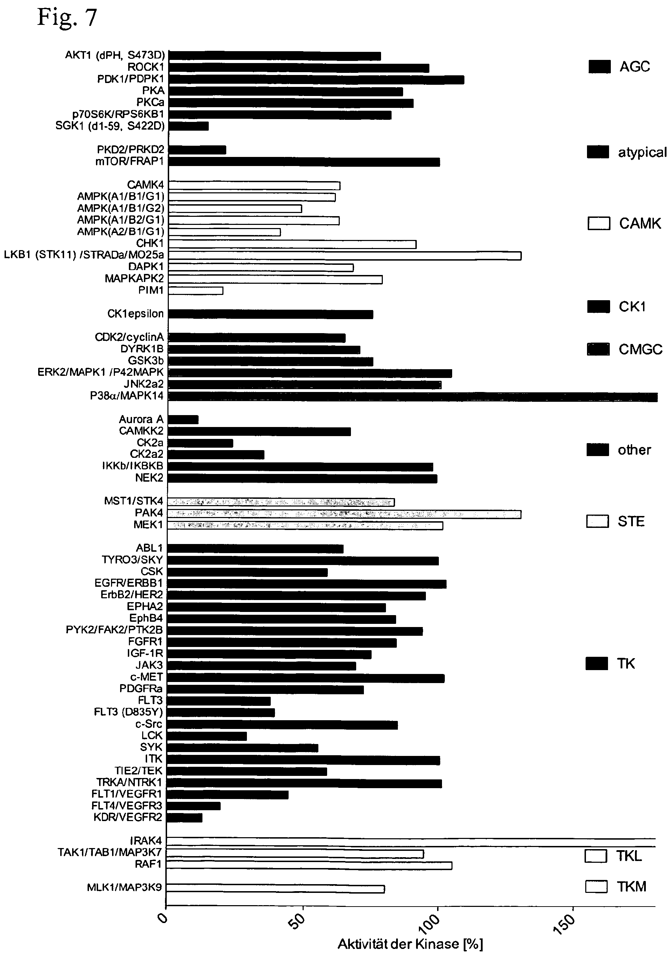

Fig. 7 zeigt das Selektivitätsprofil von TF mit einer Auswahl von 63 humanen Proteinkinasen. Die aufgelisteten PK wurden mit 10 µM TF und 10 µM ATP inkubiert. Anschließend wurde die relative prozentuale Kinaseaktivität bestimmt (Mittelwert aus n=2). 100 % Aktivität wurde als Aktivität der jeweiligen Kinase in Abwesenheit von TF definiert. Die Gruppierung der PK erfolgte nach Manning *et al.* (2002).

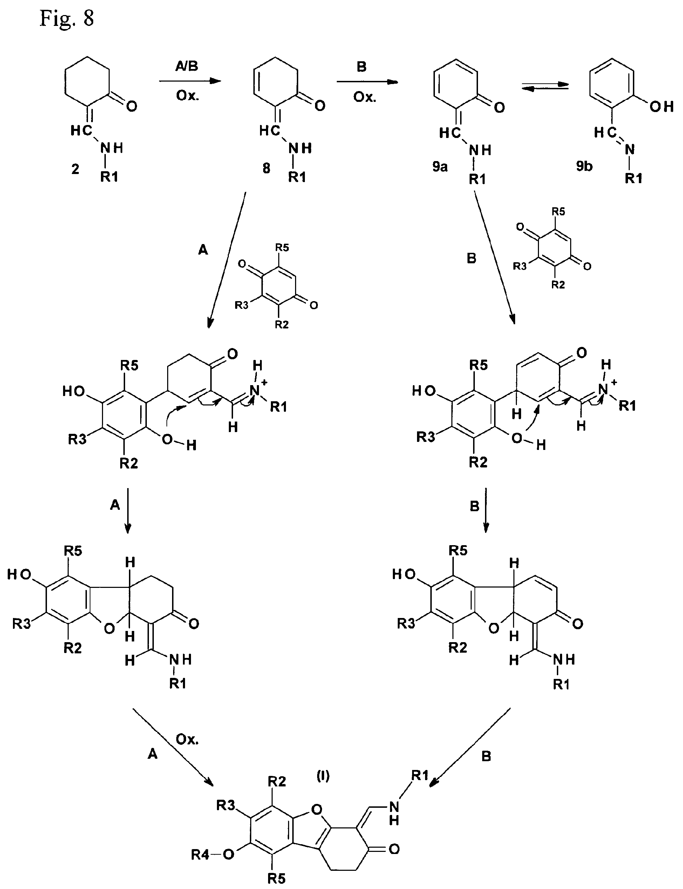

Verbindungen der allgemeinen Formel I lassen sich gemäß des in Fig. 8 gezeigten Syntheseschemas ausgehend von geeigneten 1,4-Benzoquinon-Derivaten durch Umsetzung mit entsprechenden (Aminomethylen)cyclohexanon-Derivaten gewinnen. Bei der Bildung der erfindungsgemäßen Verbindungen der allgemeinen Formel I können grundsätzlich die beiden Reaktionswege A und B beschritten werden. Aufgrund der stärkeren Oxidationskraft der chlorierten Chinone 1a und b kommt es im Gegensatz zu dem einfachen 1,4-Benzochinon bzw. 2-Methyl-1,4-benzochinon vor der Addition zu einer Dehydrierung des Enaminons 2. Die Ausbeute an Dibenzofuranon der allgemeinen Formel I kann sukzessive durch Erhöhung der Chinon-Menge von 10 % auf 20 % gesteigert werden.

Nach der Identifizierung von Ric152 als potentem CK2-Inhibitor wurden weitere Benzofuranderivate von Prof. Kuckländer zur Verfügung gestellt (Kuckländer und Töberich, 1982; Bollig, 2007). Die Wirkung dieser Substanzen auf die CK2-Aktivität wurde zunächst wieder bei einer Konzentration von 10 µM untersucht. Bei einer Hemmung der CK2-Aktivität von > 50 % wurde zusätzlich der IC₅₀ Wert der Substanz bestimmt, um die inhibitorische Potenz genauer zu quantifizieren. Die mit dem kapillarelektrophoretischen Testverfahren erhaltenen Ergebnisse der untersuchten Substanzen sind in
Tabelle 2 zusammengefasst.

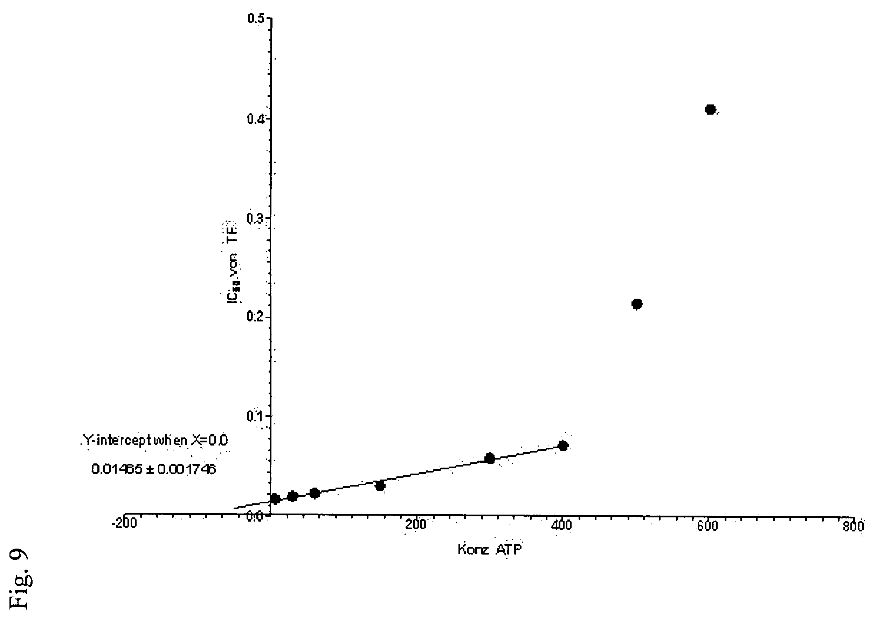

Fig. 9 die Untersuchung des Inhibitionsmodus der Verbindung TF. Dazu wurden IC₅₀-Werte von TF bei jeweils 8 verschiedenen ATP Konzentrationen bestimmt. Ein Ansteigen des IC₅₀ Wertes bei steigender ATP Konzentration deutet darauf hin, dass es sich bei TF um einen ATP-kompetitiven Inhibitor handelt. Aus diesen Daten konnte außerdem die Inhibitionskonstante Kᵢ gewonnen werden. Diese lässt sich in Fig. 9 als Schnittpunkt der Ausgleichsgerade des linearen Bereichs als Y-Achsenabschnitt ablesen. Der Kᵢ-Wert für TF beträgt demnach 14,65 nM (± 1,75nM).

**Tabelle 2: Untersuchung verschiedener Benzofuranderivate auf Hemmung der CK2 im kapillarelektrophoretischen Aktivitätstest.**

| **Substanz** | **Strukturformel** | **CK2-Aktivität bei 10 µM** | **IC**₅₀ **Wert** |
|---|---|---|---|
| TF | 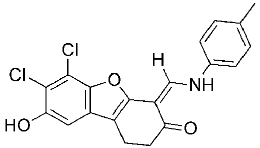 | < 4 %¹ | 0,03 µM |
| Ric151 | 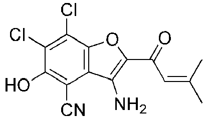 | < 4 %¹ | 0,2 µM |
| Ric152 | 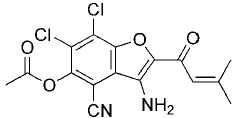 | 7 % | 0,2 µM |
| Ric138 | 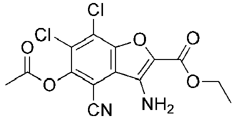 | 31 % | 2,4 µM |
| Ric149 | 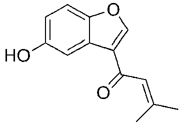 | 61 % | - |
| RicFur | 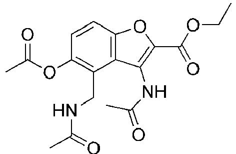 | 110 % | - |

| | | | |
|---|---|---|---|
| ¹Werte unter 4 % konnten nicht mehr verlässlich quantifiziert werden. | | | |

Die untersuchten Benzofuranderivate wiesen deutliche Unterschiede in der CK2-Hemmung auf. Ric149 und RicFur zeigten eine geringe, bzw. keine inhibitorische Wirkung auf die CK2-Aktivität. Im Gegensatz dazu führte die Hemmwirkung von TF, Ric151, Ric152, Ric138 zu einer CK2-Restaktivität von unter 50 %. Für diese Substanzen wurde zusätzlich IC₅₀ Werte bestimmt. Bei näherer Betrachtung lässt sich für die Substanzen Ric138 und Ric152 eine interessante Struktur-Wirkungsbeziehung aufzeigen. Sie unterscheiden sich lediglich durch die Seitenkette an Position 2 des Benzofuranrings. Dieser strukturelle Unterschied spiegelte sich in einem 10-fachen Unterschied des IC₅₀ Wertes wider, der für Ric138 2,4 µM und für Ric152 0,2 µM betrug. Der Austausch der Ethylesterfunktionalität von Ric138 durch das α, β-ungesättigte Carbonylsystem (3-Methylbutenon) von Ric152 wirkte sich demnach deutlich positiv auf die inhibitorische Potenz aus. Die Acetylierung der phenolischen Hydroxylgruppe in Position 5 des Benzofuranrings scheint für die Wirkung von Ric152 nicht essentiell zu sein, da das entsprechend deacetylierte Derivat Ric151 mit 0,2 µM den identischen IC₅₀ Wert aufwies. Den besten IC₅₀ Wert zeigte die als "Töberichfuran" oder kurz TF bezeichnete Verbindung (*Z*)-6,7-Dichlor-1,4-dihydro-8-hydroxy-4-[(4-methylphenylamino)methylen]-dibenzo[b,d]furan-3(2*H*)-on (Kuckländer und Töberich, 1982). Dieser lag mit 0,03 µM noch um knapp eine Zehnerpotenz unter dem IC₅₀ Wert von Ric151 und Ric152 (Figur 3).

TF wurde als äußerst potenter CK2-Inhibitor identifiziert und wurde auf seine Membrangängigkeit, wachstumshemmenden Eigenschaften und Hemmung der nativen CK2 in der humanen Prostatakarzinomzelllinie LNCaP (Horoszewicz *et al.,* 1980) untersucht. Alle Zellkulturexperimente wurden von Claudia Götz in der Arbeitsgruppe von Prof. Montenarh (Medizinische Biochemie und Molekularbiologie, Universitätskliniken des Saarlandes, Homburg/Saar) durchgeführt.

Mit einem MTT-Test wurde die Wirkung von TF auf die Viabilität von LNCaP-Zellen bestimmt. Dazu wurde diese Zelllinien mit drei TF-Konzentrationen (25 µM, 50 µM, 75 µM) behandelt. Der Effekt wurde nach 24 h, 48 h und 72 h als relative Viabilität im Vergleich zu unbehandelten Zellen bestimmt. Analog dazu wurden LNCaP-Zellen mit denselben Konzentrationen des Inhibitors TBB inkubiert, um vergleichende Aussagen unter gleichen experimentellen Bedingungen treffen zu können. Nach Behandlung der LNCaP-Zellen mit TF waren sowohl Effekte durch Steigerung der Konzentration, als auch durch Verlängerung der Inkubationsdauer zu verzeichnen. Nach 24-stündiger Inkubation mit 25 µM TF ging die Viabilität bereits auf 45 % zurück und konnte nach derselben Inkubationsdauer mit 50 µM TF noch weiter auf 30 % gesenkt werden. Die Inkubation mit 75 µM TF hatte jedoch keine weitere Reduktion zur Folge (31 % Viabilität). Die Effekte nach 24 h konnten durch Verlängerung der Inkubationsdauer jeweils verstärkt werden. So war nach 48-stündiger Inkubation mit 25 µM, 50 µM und 75 µM TF noch eine Viabilität von 37 %, 15 %, bzw. 12 % feststellbar. Nach 72-stündiger Inkubation mit einer TF-Konzentration von 25 µM war nur noch eine Viabilität von 14 % detektierbar, die durch die höheren TF-Konzentrationen von 50 µM und 75 µM noch auf 5 %, bzw. 3 % gesenkt wurde (Figur 4 A). Die Behandlung von LNCaP-Zellen mit TBB hatte einen ähnlichen Effekt, der jedoch erst bei höheren Konzentrationen, bzw. längerer Inkubationsdauer einsetzte. So hatte eine Inkubationsdauer von 24 h nur geringe Auswirkungen. Mit der maximalen TBB-Konzentration von 75 µM wurde nach 24 h noch 90 % Viabilität gemessen. Auch nach 72 h waren bei der niedrigsten TBB-Konzentration von 25 µM keine Effekte zu sehen. Erst bei der höchsten Konzentration von 75 µM war nach 48 h eine Reduktion auf 45 % und nach 72 h noch eine Viabilität von 17% zu verzeichnen (Figur 5 B). Unter Berücksichtigung der jeweiligen Inkubationsdauer wurden die Effekte der höchsten untersuchten TBB-Konzentration (75 µM) bereits von der kleinsten untersuchten TF-Konzentration (25 µM) übertroffen. Zusammenfassend konnte festgestellt werden, dass die Viabilität von LNCaP-Zellen durch beide Substanzen herabgesetzt wurde. Der neu identifizierte CK2-Inhibitor TF wirkte jedoch deutlich potenter als der schon bekannte Inhibitor TBB.

Anschließend wurde untersucht, ob die TF-vermittelte Reduktion der Zellviabilität auf apoptotische Ereignisse zurückgeführt werden konnte. Zu diesem Zweck wurde die Spaltung von Poly-(ADP-Ribose)-Polymerase (PARP) in den Zellen nachgewiesen. Caspase 3 spaltet die 115 kDa große PARP in zwei stabile Fragmente: die 24 kDa große, DNA-bindende, N-terminale Domäne und das 89 kDa große, C-terminale Fragment. Diese spezifische Proteolyse von PARP kennzeichnet den irreversiblen Punkt der Einleitung der Apoptose und kann im Western Blot von Zelllysaten mit einem spezifischen PARP-Antikörper nachgewiesen werden. LNCaP-Zellen wurden auf PARP-Spaltung nach 24- und 48-stündiger Inkubation mit 25 µM, 50 µM und 75 µM TF untersucht und mit dem Effekt von TBB gleicher Konzentration verglichen. Nach einer Inkubation mit TF war ab 50 µM zu sehen, dass die Bande des 89 kDa PARP-Fragments konzentrationsabhängig an Intensität zunahm. Dieser Effekt war sowohl nach einer Inkubationsdauer von 24 h, als auch, in verstärkter Form, nach 48 h zu erkennen. Eine Behandlung der LNCaP-Zellen mit TBB führte in diesen Experimenten zu nahezu identischen Ergebnissen (Figur 6). Hier war auch schon bei einer Konzentration von 25 µM eine leichte Bande bei 89 kDa zu erkennen. LNCaP-Zellen, die mit 75 µM TBB inkubiert wurden, konnten aufgrund technischer Schwierigkeiten nicht ausgewertet werden. Als Ergebnis bleibt festzustellen, dass sowohl TF als auch TBB in LNCaP-Zellen im untersuchten Konzentrationsbereich ähnlich starke pro-apoptotische Wirkung zeigen.

LNCaP-Zellen wurden analog zu den oben beschriebenen Experimenten mit TF inkubiert. Nach 24 h oder 48 h wurde die CK2-Aktivität von Lysaten mit Hilfe des radiometrischen CK2-Tests gemessen. Diese Untersuchung gab Aufschluss darüber, ob die am isolierten Enzym gefundene Hemmung von TF im zellulären System bestätigt werden konnte. Als Vergleich diente der Einfluss der jeweils gleichen Konzentration von TBB auf die Hemmung der zellulären CK2-Aktivität. Zellen wurden mit 25 µM, 50 µM oder 75 µM TF für 24 h oder 48 h inkubiert, danach gewaschen und lysiert. Von diesen Zelllysaten wurde die relative CK2-Aktivität berechnet. Als Bezug diente die CK2-Aktivität der Zelllysate von unbehandelten und entsprechend lange inkubierten Zellen. Um eventuell auftretende Schwankungen im radiometrischen Test auszugleichen, wurden im Falle von TF je zwei Proben pro Parameter gemessen. Die TBB-Proben wurden als qualitative Kontrolle jeweils einzeln angesetzt und gemessen. Beide Substanzen führten ab einer Konzentration von 25 µM zu einer Reduktion der endogenen CK2-Aktivität von LNCaP-Zellen auf 50 %. Durch eine Steigerung der Konzentration konnte in keinem Fall eine weitere Verminderung der CK2-Aktivität verzeichnet werden (Figur 6).

Im Gegensatz zum MTT-Test war in keiner Versuchsreihe ein Zusammenhang zwischen verlängerter Inkubationsdauer mit der jeweiligen Substanz und einer verstärkten Hemmung der endogenen CK2-Aktivität zu erkennen. Parallel zum Aktivitätstest wurde durch Western Blots nachgewiesen, dass die exprimierte Menge an endogener CK2α in keinem der Zelllysate reduziert war. Die beobachtete Abnahme der CK2-Aktivität ist demnach auf die Modulation der CK2-Enzymaktivität zurückzuführen. Wegen der Schwankungen des radiometrischen Tests und der geringen Anzahl an gleichen Ansätzen sollten diese Ergebnisse jedoch in erster Linie als qualitative Untersuchung gewertet werden. So stellte z. B. die Probe der 24 h mit 75 µM TBB inkubierten LNCaP-Zellen mit einer CK2-Aktivität von 90 % höchstwahrscheinlich einen Ausreißer dar. Zusammengenommen konnte gezeigt werden, dass TF die endogene CK2-Aktivität in LNCaP-Zellen senkt. Die Wirkstärke von TF lag in etwa auf dem Niveau von TBB. Der Nachweis der Hemmung intrazellulärer CK2 belegt darüber hinaus die Fähigkeit von TF zur Zellpermeation.

Nachdem die inhibitorische Potenz von TF in LNCaP-Zellen bestätigt werden konnte, sollte untersucht werden, ob TF einen selektiven CK2-Inhibitor darstellt. Dazu wurde von dem Unternehmen "Reaction Biology" (Malvern, PA, USA) die Hemmwirkung von TF auf 63 isolierte humane Proteinkinasen (PK) untersucht. Darunter waren PK aus allen Hauptgruppen des humanen Kinoms (Manning *et al.,* 2002) vertreten. Die Ergebnisse dieser Untersuchung sind in Figur 8, geordnet nach den Hauptgruppen humaner PK, dargestellt. In dieser Testreihe wurden alle PK mit je 10 µM ATP und 10 µM TF inkubiert. In einem anschließenden radiometrischen Test wurde die jeweilige Kinaseaktivität in Bezug auf eine Probe ohne TF bestimmt. Da sich die K_{M}-Werte der PK bzgl. ATP teilweise deutlich unterscheiden (Knight und Shokat, 2005), ermöglichen die Ergebnisse lediglich einen semi-quantitativen Vergleich der Inhibition durch TF. Definiert man Hemmung in diesem Test als eine Aktivitätsminderung > 50 %, so wurden neben CK2α und CK2α' zwölf der 63 PK von TF gehemmt und 49 der 63 PK reagierten schwach, überhaupt nicht oder zeigten im Fall von IRAK4, MAPK14, PAK4 oder LBK1 eine deutliche Steigerung der Aktivität. CK2α (von "Reaction Biology" als "CK2a" bezeichnet) und CK2α' (von "Reaction Biology" als "CK2a2" bezeichnet) wurde in diesem Test mit knapp 24 %, bzw. 35 % Restaktivität (Figur 7) schwächer inhibiert, als dies in der kapillarelektrophoretischen Inhibitionsuntersuchung mit dem aufgereinigten CK2-Holoenzym (< 4 % Restaktivität,

Tabelle 2) festgestellt wurde. Dabei müssen jedoch auch die unterschiedlichen Testparameter, wie Substrat- und ATP-Konzentrationen, Puffer, etc. berücksichtigt werden, sodass ein direkter Vergleich prozentualer Hemmdaten an dieser Stelle nicht möglich ist. Die sieben PK, die am stärksten (>70 %) gehemmt wurden, sind in Tabelle 3 separat aufgelistet. Obwohl diese Ergebnisse lediglich einen groben Überblick über das quantitative inhibitorische Potential von TF gaben, deuteten sie darauf hin, dass TF ein multiselektiver Proteinkinaseinhibitor ist.

**Tabelle 3: Humane Proteinkinasen, die von TF zu mindestens 30 % gehemmt wurden.**

| **Proteinkinase** | **Aktivität** | **UniProt Accession #** | **Physiologische Funktion** |
|---|---|---|---|
| Aurora A | 11,00% | 014965 | Zellzyklusregulation |
| KDR/VEGFR2 | 13,05 % | P35968 | Gefäßentwicklung, Angiogenese |
| SGK1 (d1-59, S422D) | 14,16 % | 000141 | Stressantwort, Zellüberleben |
| FLT4/VEGFR3 | 19,68 % | P35916 | Zellproliferation |
| PIM1 | 19,90 % | P11309 | Zellproliferation, Zellüberleben |
| PKD2/PRKD2 | 20,90 % | Q9BZL6 | Resistenz gegen oxidativen Stress |
| LCK | 29,20 % | P06239 | T-Zell-Differenzierung |

### Literaturverzeichnis

Ahmad, K.A., Wang, G., Unger, G., Slaton, J. und Ahmed, K. (2008) Protein kinase CK2-a key suppressor of apoptosis. Adv Enzyme Regul 48:179-87.
Ahmed, K., Issinger, O.-G., R. Marshak, D. und Pyerin, W. (1994) Editorial. Cell Mol Biol Res 40:371-2.
Allende, J.E. und Allende, C.C. (1995) Protein kinase CK2: an enzyme with multiple substrates and a puzzling regulation. FASEB J 9:313-23.
Allende-Vega, N., Dias, S., Milne, D. und Meek, D. (2005) Phosphorylation of the acidic domain of Mdm2 by protein kinase CK2. Mol Cell Biochem 274:85-90.
Augustine, S.A.J., Kleshchenko, Y.Y., Nde, P.N., Pratap, S., Ager, E.A., Burns, J.M., Lima, M.F. und Villalta, F. (2006) Molecular cloning of a Trypanosoma cruzi cell surface casein kinase II substrate, Tc-1, involved in cellular infection. Infect Immun 74:3922-9.
Barz, T., Ackermann, K., Dubois, G., Eils, R. und Pyerin, W. (2003) Genome-wide expression screens indicate a global role for protein kinase CK2 in chromatin remodeling. J Cell Sci 116:1563-77.
Battistutta, R. (2009) Protein kinase CK2 in health and disease: Structural bases of protein kinase CK2 inhibition. Cell Mol Life Sci 66:1868-89.
Battistutta, R., Sarno, S., De Moliner, E., Papinutto, E., Zanotti, G. und Pinna, L.A. (2000) The replacement of ATP by the competitive inhibitor emodin induces conformational modifications in the catalytic site of protein kinase CK2. J Biol Chem 275:29618-22.
Bibby, A.C. und Litchfield, D.W. (2005) The multiple personalities of the regulatory subunit of protein kinase CK2: CK2 dependent and CK2 independent roles reveal a secret identity for CK2beta. Int J Biol Sci 1:67-79.
Blanquet, P.R. (2000) Casein kinase 2 as a potentially important enzyme in the nervous system. Prog Neurobiol 60:211-46.
Blume-Jensen, P. und Hunter, T. (2001) Oncogenic kinase signalling. Nature 411:355-65.
Bollig, R. (2007) Neue cytostatisch wirksame Reaktionsprodukte der erweiterten Nenitzescu-Reaktion. Dissertation, Heinrich-Heine-Universität, Düsseldorf.
Bortolato, A., Cozza, G. und Moro, S. (2008) Protein kinase CK2 inhibitors: emerging anticancer therapeutic agents? Anti-Cancer Agents Med Chem 8:798-806.
Bretner, M., Najda-Bernatowicz, A., Lebska, M., Muszyńska, G., Kilanowicz, A. und Sapota, A. (2008) New inhibitors of protein kinase CK2, analogues of benzimidazole and benzotriazole. Mol Cell Biochem 316:87-9.
Brown, C.J., Lain, S., Verma, C.S., Fersht, A.R. und Lane, D.P. (2009) Awakening guardian angels: drugging the p53 pathway. Nat Rev Cancer 9:862-73.
Buchou, T., Vernet, M., Blond, O., Jensen, H.H., Pointu, H., Olsen, B.B., Cochet, C., Issinger, O.-G. und Boldyreff, B. (2003) Disruption of the regulatory beta subunit of protein kinase CK2 in mice leads to a cell-autonomous defect and early embryonic lethality. Mol Cell Biol 23:908-15.
Burnett, G. und Kennedy, E.P. (1954) The enzymatic phosphorylation of proteins. J Biol Chem 211:969-80.
Caples, M.J., Clements, J.E. und Barber, S.A. (2006) Protein kinase CK2 phosphorylates the Nef protein from a neurovirulent simian immunodeficiency virus. Virology 348:156-64.
Carpenter, G., King, L. und Cohen, S. (1979) Rapid enhancement of protein phosphorylation in A-431 cell membrane preparations by epidermal growth factor. J Biol Chem 254:4884-91.
Chalhoub, N. und Baker, S.J. (2009) PTEN and the PI3-kinase pathway in cancer. Annu Rev Pathol 4:127-50.
Cheek, S., Ginalski, K., Zhang, H. und Grishin, N.V. (2005) A comprehensive update of the sequence and structure classification of kinases. BMC Struct Biol 5:6.
Cheek, S., Zhang, H. und Grishin, N.V. (2002) Sequence and structure classification of kinases. J Mol Biol 320:855-81.
Chen, J., Gao, C., Shi, Q., Wang, G., Lei, Y.-J., Shan, B., Zhang, B., Dong, C., Shi, S., Wang, X., Tian, C., Han, J. und Dong, X.-P. (2008) Casein kinase II interacts with prion protein in vitro and forms complex with native prion protein in vivo. Acta Biochim Biophys Sin (Shanghai) 40:1039-47.
Chien, W.M., Parker, J.N., Schmidt-Grimminger, D.C., Broker, T.R. und Chow, L.T. (2000) Casein kinase II phosphorylation of the human papillomavirus-18 E7 protein is critical for promoting S-phase entry. Cell Growth Differ 11:425-35.
Chua, P., Pierre, F. und Whitten, J.P. (2008) Serine-threonine protein kinase and PARP modulators. International Patent Nummer: WO 2008/028168
Cohen, P. (1999) The development and therapeutic potential of protein kinase inhibitors. Curr Opin Chem Biol 3:459-65.
Cohen, P. (2002a) The origins of protein phosphorylation. Nat Cell Biol 4:E127-30.
Cohen, P. (2002b) Protein kinases--the major drug targets of the twenty-first century? Nat Rev Drug Discov 1:309-15.
Cozza, G., Bonvini, P., Zorzi, E., Poletto, G., Pagano, M.A., Sarno, S., Donella-Deana, A., Zagotto, G., Rosolen, A., Pinna, L.A., Meggio, F. und Moro, S. (2006) Identification of ellagic acid as potent inhibitor of protein kinase CK2: a successful example of a virtual screening application. J Med Chem 49:2363-6.
Cozza, G., Bortolato, A. und Moro, S. (2010) How druggable is protein kinase CK2? Med Res Rev 30:419-62.
Cozza, G., Mazzorana, M., Papinutto, E., Bain, J., Elliott, M., di Maira, G., Gianoncelli, A., Pagano, M.A., Sarno, S., Ruzzene, M., Battistutta, R., Meggio, F., Moro, S., Zagotto, G. und Pinna, L.A. (2009) Quinalizarin as a potent, selective and cell-permeable inhibitor of protein kinase CK2. Biochem J 421:387-95.
Dancey, J.E. (2009) Kinase inhibitor 4 minisymposium summary. Expert Rev Anticancer Ther 9:891-4.
Daya-Makin, M., Sanghera, J.S., Mogentale, T.L., Lipp, M., Parchomchuk, J., Hogg, J.C. und Pelech, S.L. (1994) Activation of a tumor-associated protein kinase (p40TAK) and casein kinase 2 in human squamous cell carcinomas and adenocarcinomas of the lung. Cancer Res 54:2262-8.
Deana, A.D., Meggio, F., Pinna, L.A. und Moret, V. (1978) Different susceptibility of whole casein components to enzymatic phosphorylation by two forms of rat liver 'casein kinase'. Biochim Biophys Acta 524:316-26.
Delorme, V., Cayla, X., Faure, G., Garcia, A. und Tardieux, I. (2003) Actin dynamics is controlled by a casein kinase II and phosphatase 2C interplay on Toxoplasma gondii Toxofilin. Mol Biol Cell 14:1900-12.
DePaoli-Roach, A.A., Roach, P.J., Pham, K., Kramer, G. und Hardesty, B. (1981) Phosphorylation of glycogen synthase and of the beta subunit of eukaryotic initiation factor two by a common protein kinase. J Biol Chem 256:8871-4.
Desagher, S., Osen-Sand, A., Montessuit, S., Magnenat, E., Vilbois, F., Hochmann, A., Journot, L., Antonsson, B. und Martinou, J.C. (2001) Phosphorylation of bid by casein kinases I and II regulates its cleavage by caspase 8. Mol Cell 8:601-11.
Di Maira, G., Brustolon, F., Pinna, L.A. und Ruzzene, M. (2009) Dephosphorylation and inactivation of Akt/PKB is counteracted by protein kinase CK2 in HEK 293T cells. Cell Mol Life Sci 66:3363-73.
Di Maira, G., Salvi, M., Arrigoni, G., Marin, O., Sarno, S., Brustolon, F., Pinna, L.A. und Ruzzene, M. (2005) Protein kinase CK2 phosphorylates and upregulates Akt/PKB. Cell Death Differ 12:668-77.
Dominguez, I., Sonenshein, G.E. und Seldin, D.C. (2009) Protein kinase CK2 in health and disease: CK2 and its role in Wnt and NF-kappaB signaling: linking development and cancer. Cell Mol Life Sci 66:1850-7.
Druker, B.J. (2009) Perspectives on the development of imatinib and the future of cancer research. Nat Med 15:1149-52.
Eglen, R.M. und Reisine, T. (2009) The current status of drug discovery against the human kinome. Assay Drug Dev Technol 7:22-43.
Escalier, D., Silvius, D. und Xu, X. (2003) Spermatogenesis of mice lacking CK2alpha': failure of germ cell survival and characteristic modifications of the spermatid nucleus. Mol Reprod Dev 66:190-201.
Farah, M., Parhar, K., Moussavi, M., Eivemark, S. und Salh, B. (2003) 5,6-Dichlororibifuranosylbenzimidazole- and apigenin-induced sensitization of colon cancer cells to TNF-alpha-mediated apoptosis. Am J Physiol Gastrointest Liver Physiol 285:G919-28.
Faust, M. und Montenarh, M. (2000) Subcellular localization of protein kinase CK2. A key to its function? Cell Tissue Res 301:329-40.
Faust, R.A., Niehans, G., Gapany, M., Hoistad, D., Knapp, D., Cherwitz, D., Davis, A., Adams, G.L. und Ahmed, K. (1999) Subcellular immunolocalization of protein kinase CK2 in normal and carcinoma cells. Int J Biochem Cell Biol 31:941-9.
Filhol, O. und Cochet, C. (2009) Protein kinase CK2 in health and disease: Cellular functions of protein kinase CK2: a dynamic affair. Cell Mol Life Sci 66:1830-9.
Fischer, E.H. (1993) Protein Phosphorylation and Cellular Regulation II (Nobel Lecture). Angew Chem, Int Ed Engl 32:1130-7.
Fu, L. und Lee, C.C. (2003) The circadian clock: pacemaker and tumour suppressor. Nat Rev Cancer 3:350-61.
Glover, C.V. (1998) On the physiological role of casein kinase II in Saccharomyces cerevisiae. Prog Nucleic Acid Res Mol Biol 59:95-133.
Graham, K.C. und Litchfield, D.W. (2000) The regulatory beta subunit of protein kinase CK2 mediates formation of tetrameric CK2 complexes. J Biol Chem 275:5003-10.
Graziani, Y., Erikson, E. und Erikson, R.L. (1983) Characterization of the Rous sarcoma virus transforming gene product. J Biol Chem 258:6344-51.
Guerra, B. (2006) Protein kinase CK2 subunits are positive regulators of AKT kinase. Int J Oncol 28:685-93.
Guerra, B. und Issinger, O.-G. (1999) Protein kinase CK2 and its role in cellular proliferation, development and pathology. Electrophoresis 20:391-408.
Guerra, B. und Issinger, O.-G. (2008) Protein kinase CK2 in human diseases. Curr Med Chem 15:1870-86.
Hanahan, D. und Weinberg, R.A. (2000) The hallmarks of cancer. Cell 100:57-70.
Harvey, E.J., Li, N. und Ramji, D.P. (2007) Critical role for casein kinase 2 and phosphoinositide-3-kinase in the interferon-gamma-induced expression of monocyte chemoattractant protein-1 and other key genes implicated in atherosclerosis. Arterioscler Thromb Vasc Biol 27:806-12.
Hastie, C.J., McLauchlan, H.J. und Cohen, P. (2006) Assay of protein kinases using radiolabeled ATP: a protocol. Nat Protoc 1:968-71.
Hidaka, H., Inagaki, M., Kawamoto, S. und Sasaki, Y. (1984) Isoquinolinesulfonamides, novel and potent inhibitors of cyclic nucleotide dependent protein kinase and protein kinase C. Biochemistry 23:5036-41.
Homma, M.K. und Homma, Y. (2008) Cell cycle and activation of CK2. Mol Cell Biochem 316:49-55.
Hora, R., Bridges, D.J., Craig, A. und Sharma, A. (2009) Erythrocytic casein kinase II regulates cytoadherence of Plasmodium falciparum-infected red blood cells. J Biol Chem 284:6260-9.
Horoszewicz, J.S., Leong, S.S., Chu, T.M., Wajsman, Z.L., Friedman, M., Papsidero, L., Kim, U., Chai, L.S., Kakati, S., Arya, S.K. und Sandberg, A.A. (1980) The LNCaP cell line-a new model for studies on human prostatic carcinoma. Prog Clin Biol Res 37:115-32.
Hung, M.-S., Xu, Z., Lin, Y.-C., Mao, J.-H., Yang, C.-T., Chang, P.-J., Jablons, D.M. und You, L. (2009) Identification of hematein as a novel inhibitor of protein kinase CK2 from a natural product library. BMC Canc 9:135.
Jia, Y., Quinn, C.M., Kwak, S. und Talanian, R.V. (2008) Current in vitro kinase assay technologies: the quest for a universal format. Curr Drug Discov Technol 5:59-69.
Kelliher, M.A., Seldin, D.C. und Leder, P. (1996) Tal-1 induces T cell acute lymphoblastic leukemia accelerated by casein kinase II alpha. EMBO J 15:5160-6.
Kennedy, E. und Smith, S. (1954) The isolation of radioactive phosphoserine from phosphoprotein of the Ehrlich ascites tumor. J Biol Chem 207:153-63.
Kim, J.S., Eom, J.I., Cheong, J.-W., Choi, A.J., Lee, J.K., Yang, W.I. und Min, Y.H. (2007) Protein kinase CK2alpha as an unfavorable prognostic marker and novel therapeutic target in acute myeloid leukemia. Clin Cancer Res 13:1019-28.
Klumpp, S. und Krieglstein, J. (2005) Reversible phosphorylation of histidine residues in vertebrate proteins. Biochim Biophys Acta 1754:291-5.
Knight, Z.A. und Shokat, K.M. (2005) Features of selective kinase inhibitors. Chem Biol 12:621-37.
Kramerov, A.A., Saghizadeh, M., Caballero, S., Shaw, L.C., Li Calzi, S., Bretner, M., Montenarh, M., Pinna, L.A., Grant, M.B. und Ljubimov, A.V. (2008) Inhibition of protein kinase CK2 suppresses angiogenesis and hematopoietic stem cell recruitment to retinal neovascularization sites. Mol Cell Biochem 316:177-86.
Krebs, E.G. (1993) Protein Phosphorylation and Cellular Regulation I (Nobel Lecture). Angew Chem, Int Ed Engl 32:1122-9.
Krebs, E.G. und Fischer, E. (1956) The phosphorylase b to a converting enzyme of rabbit skeletal muscle. Biochim Biophys Acta 20:150-7.
Kuckländer, U. und Töberich, H. (1982) Zur Umsetzung von 2-(Aminomethylen)cyclohexanon-Derivaten mit Dichlorchinonen. Chem Ber 116:152-8.
Kuenzel, E.A., Mulligan, J.A., Sommercorn, J. und Krebs, E.G. (1987) Substrate specificity determinants for casein kinase II as deduced from studies with synthetic peptides. J Biol Chem 262:9136-40.
Landesman-Bollag, E., Channavajhala, P.L., Cardiff, R.D. und Seldin, D.C. (1998) p53 deficiency and misexpression of protein kinase CK2alpha collaborate in the development of thymic lymphomas in mice. Oncogene 16:2965-74.
Landesman-Bollag, E., Romieu-Mourez, R., Song, D.H., Sonenshein, G.E., Cardiff, R.D. und Seldin, D.C. (2001a) Protein kinase CK2 in mammary gland tumorigenesis. Oncogene 20:3247-57.
Landesman-Bollag, E., Song, D.H., Romieu-Mourez, R., Sussman, D.J., Cardiff, R., Sonenshein, G. und Seldin, D.C. (2001b) Protein kinase CK2: signaling and tumorigenesis in the mammary gland. Mol Cell Biochem 227:153-65.
Lane, D.P. (1992) Cancer. p53, guardian of the genome. Nature 358:15-6.
Laramas, M., Pasquier, D., Filhol, O., Ringeisen, F., Descotes, J.-L. und Cochet, C. (2007) Nuclear localization of protein kinase CK2 catalytic subunit (CK2alpha) is associated with poor prognostic factors in human prostate cancer. Eur J Cancer 43:928-34.
Laudet, B., Barette, C., Dulery, V., Renaudet, O., Dumy, P., Metz, A., Prudent, R., Deshiere, A., Dideberg, O., Filhol, O. und Cochet, C. (2007) Structure-based design of small peptide inhibitors of protein kinase CK2 subunit interaction. Biochem J 408:363-73.
Laudet, B., Moucadel, V., Prudent, R., Filhol, O., Wong, Y.-S., Royer, D. und Cochet, C. (2008) Identification of chemical inhibitors of protein-kinase CK2 subunit interaction. Mol Cell Biochem 316:63-9.
Li, C., Liu, X., Lin, X. und Chen, X. (2009) Structure-activity relationship of 7 flavonoids on recombinant human protein kinase CK2 holoenzyme. J Cent S Univ Med Sci 34:20-6.
Li, P.-F., Li, J., Müller, E.-C., Otto, A., Dietz, R. und von Harsdorf, R. (2002) Phosphorylation by protein kinase CK2: a signaling switch for the caspase-inhibiting protein ARC. Mol Cell 10:247-58.
Lin, J., Kilman, V.L., Keegan, K., Paddock, B., Emery-Le, M., Rosbash, M. und Allada, R. (2002) A role for casein kinase 2alpha in the Drosophila circadian clock. Nature 420:816-20.
Lipinski, C.A., Lombardo, F., Dominy, B. und Feeney, P. (2001) Experimental and computational approaches to estimate solubility and permeability in drug discovery and development settings. Adv Drug Del Rev 46:3-26.
Litchfield, D.W. (2003) Protein kinase CK2: structure, regulation and role in cellular decisions of life and death. Biochem J 369:1-15.
Litchfield, D.W., Bosc, D., Canton, D.A., Saulnier, R.B., Vilk, G. und Zhang, C. (2001) Functional specialization of CK2 isoforms and characterization of isoform-specific binding partners. Mol Cell Biochem 227:21-9.
Llobet, D., Eritja, N., Encinas, M., Llecha, N., Yeramian, A., Pallares, J., Sorolla, A., Gonzalez-Tallada, F.J., Matias-Guiu, X. und Dolcet, X. (2008) CK2 controls TRAIL and Fas sensitivity by regulating FLIP levels in endometrial carcinoma cells. Oncogene 27:2513-24.
Lorenz, P., Pepperkok, R. und Pyerin, W. (1994) Requirement of casein kinase 2 for entry into and progression through early phases of the cell cycle. Cell Mol Biol Res 40:519-27.
Lou, D.Y., Dominguez, I., Toselli, P., Landesman-Bollag, E., O'Brien, C. und Seldin, D.C. (2008) The alpha catalytic subunit of protein kinase CK2 is required for mouse embryonic development. Mol Cell Biol 28:131-9.
Lozeman, F.J., Litchfield, D.W., Piening, C., Takio, K., Walsh, K.A. und Krebs, E.G. (1990) Isolation and characterization of human cDNA clones encoding the alpha and the alpha' subunits of casein kinase II. Biochemistry 29:8436-47.
Ma, H., Deacon, S. und Horiuchi, K. (2008) The challenge of selecting protein kinase assays for lead discovery optimization. Expert Opin Drug Discov 3:607-21.
Maier, B., Wendt, S., Vanselow, J.T., Wallach, T., Reischl, S., Oehmke, S., Schlosser, A. und Kramer, A. (2009) A large-scale functional RNAi screen reveals a role for CK2 in the mammalian circadian clock. Genes Dev 23:708-18.
Manning, G., Whyte, D.B., Martinez, R., Hunter, T. und Sudarsanam, S. (2002) The protein kinase complement of the human genome. Science 298:1912-34.
Mantovani, A., Allavena, P., Sica, A. und Balkwill, F. (2008) Cancer-related inflammation. Nature 454:436-44.
McDonnell, M.A., Abedin, M.J., Melendez, M., Platikanova, T.N., Ecklund, J.R., Ahmed, K. und Kelekar, A. (2008) Phosphorylation of murine caspase-9 by the protein kinase casein kinase 2 regulates its cleavage by caspase-8. J Biol Chem 283:20149-58.
Medina-Palazon, C., Gruffat, H., Mure, F., Filhol, O., Vingtdeux-Didier, V., Drobecq, H., Cochet, C., Sergeant, N., Sergeant, A. und Manet, E. (2007) Protein kinase CK2 phosphorylation of EB2 regulates its function in the production of Epstein-Barr virus infectious viral particles. J Virol 81:11850-60.
Meggio, F., Boldyreff, B., Marin, O., Pinna, L.A. und Issinger, O.-G. (1992) Role of the beta subunit of casein kinase-2 on the stability and specificity of the recombinant reconstituted holoenzyme. Eur J Biochem 204:293-7.
Meggio, F. und Pinna, L.A. (2003) One-thousand-and-one substrates of protein kinase CK2? FASEB J 17:349-68.
Meggio, F., Shugar, D. und Pinna, L.A. (1990) Ribofuranosyl-benzimidazole derivatives as inhibitors of casein kinase-2 and casein kinase-1. Eur J Biochem 187:89-94.
Montenarh, M. (1997) Das Wachstumssuppressorprotein p53, seine zellulären Partner und das Prostatakarzinom. Aktuel Urol 28:371-6.
Mottet, D., Ruys, S., Demazy, C., Raes, M. und Michiels, C. (2005) Role for casein kinase 2 in the regulation of HIF-1 activity. Int J Cancer 117:764-74.
Münstermann, U., Fritz, G., Seitz, G., Lu, Y.P., Schneider, H.R. und Issinger, O.G. (1990) Casein kinase II is elevated in solid human tumours and rapidly proliferating nonneoplastic tissue. Eur J Biochem 189:251-7.
Nie, Z., Perretta, C., Erickson, P., Margosiak, S., Lu, J., Averill, A., Almassy, R. und Chu, S. (2008) Structure-based design and synthesis of novel macrocyclic pyrazolo[1,5-a] [1,3,5]triazine compounds as potent inhibitors of protein kinase CK2 and their anticancer activities. Bioorg Med Chem Lett 18:619-23.
Niefind, K., Guerra, B., Ermakova, I. und Issinger, O.-G. (2001) Crystal structure of human protein kinase CK2: insights into basic properties of the CK2 holoenzyme. EMBO J 20:5320-31.
Niefind, K. und Issinger, O.-G. (2005) Primary and secondary interactions between CK2alpha and CK2beta lead to ring-like structures in the crystals of the CK2 holoenzyme. Mol Cell Biochem 274:3-14.
Niefind, K., Pütter, M., Guerra, B., Issinger, O.-G. und Schomburg, D. (1999) GTP plus water mimic ATP in the active site of protein kinase CK2. Nat Struct Biol 6:1100-3.
ole-MoiYoi, O.K. (1995) Casein kinase II in theileriosis. Science 267:834-6.
Olsen, B.B., Bjørling-Poulsen, M. und Guerra, B. (2007) Emodin negatively affects the phosphoinositide 3-kinase/AKT signalling pathway: a study on its mechanism of action. Int J Biochem Cell Biol 39:227-37.
Olsen, B.B., Boldyreff, B., Niefind, K. und Issinger, O.-G. (2006) Purification and characterization of the CK2alpha'-based holoenzyme, an isozyme of CK2alpha: a comparative analysis. Protein Expr Purif 47:651-61.
Olsten, M.E.K. und Litchfield, D.W. (2004) Order or chaos? An evaluation of the regulation of protein kinase CK2. Biochem Cell Biol 82:681-93.
Olsten, M.E.K., Weber, J.E. und Litchfield, D.W. (2005) CK2 interacting proteins: emerging paradigms for CK2 regulation? Mol Cell Biochem 274:115-24.
Pagano, M.A., Bain, J., Kazimierczuk, Z., Sarno, S., Ruzzene, M., Di Maira, G., Elliott, M., Orzeszko, A., Cozza, G., Meggio, F. und Pinna, L.A. (2008) The selectivity of inhibitors of protein kinase CK2: an update. Biochem J 415:353-65.
Pagano, M.A., Marin, O., Cozza, G., Sarno, S., Meggio, F., Treharne, K.J., Mehta, A. und Pinna, L.A. (2010) Cystic fibrosis transmembrane regulator fragments with the Phe508 deletion exert a dual allosteric control over the master kinase CK2. Biochem J 426:19-29.
Pagano, M.A., Meggio, F., Ruzzene, M., Andrzejewska, M., Kazimierczuk, Z. und Pinna, L.A. (2004) 2-Dimethylamino-4,5,6,7-tetrabromo-1H-benzimidazole: a novel powerful and selective inhibitor of protein kinase CK2. Biochem Biophys Res Commun 321:1040-4.
Pagano, M.A., Poletto, G., Di Maira, G., Cozza, G., Ruzzene, M., Sarno, S., Bain, J., Elliott, M., Moro, S., Zagotto, G., Meggio, F. und Pinna, L.A. (2007) Tetrabromocinnamic acid (TBCA) and related compounds represent a new class of specific protein kinase CK2 inhibitors. ChemBioChem 8:129-39.
Pallares, J., Llobet, D., Santacana, M., Eritja, N., Velasco, A., Cuevas, D., Lopez, S., Palomar-Asenjo, V., Yeramian, A., Dolcet, X. und Matias-Guiu, X. (2009) CK2beta is expressed in endometrial carcinoma and has a role in apoptosis resistance and cell proliferation. Am J Pathol 174:287-96.
Perea, S.E., Reyes, O., Baladron, I., Perera, Y., Farina, H., Gil, J., Rodriguez, A., Bacardi, D., Marcelo, J.L., Cosme, K., Cruz, M., Valenzuela, C., López-Saura, P.A., Puchades, Y., Serrano, J.M., Mendoza, O., Castellanos, L., Sanchez, A., Betancourt, L., Besada, V., Silva, R., López, E., Falcón, V., Hernändez, I., Solares, M., Santana, A., Díaz, A., Ramos, T., López, C., Ariosa, J., González, L.J., Garay, H., Gómez, D., Gómez, R., Alonso, D.F., Sigman, H., Herrera, L. und Acevedo, B. (2008) CIGB-300, a novel proapoptotic peptide that impairs the CK2 phosphorylation and exhibits anticancer properties both in vitro and in vivo. Mol Cell Biochem 316:163-7.
Perea, S.E., Reyes, O., Puchades, Y., Mendoza, O., Vispo, N.S., Torrens, I., Santos, A., Silva, R., Acevedo, B., López, E., Falcón, V. und Alonso, D.F. (2004) Antitumor effect of a novel proapoptotic peptide that impairs the phosphorylation by the protein kinase 2 (casein kinase 2). Cancer Res 64:7127-9.
Perera, Y., Farina, H.G., Gil, J., Rodriguez, A., Benavent, F., Castellanos, L., Gómez, R.E., Acevedo, B.E., Alonso, D.F. und Perea, S.E. (2009) Anticancer peptide CIGB-300 binds to nucleophosmin/B23, impairs its CK2-mediated phosphorylation, and leads to apoptosis through its nucleolar disassembly activity. Mol Cancer Ther 8:1189-96.
Pinna, L.A. (1990) Casein kinase 2: an 'eminence grise' in cellular regulation? Biochim Biophys Acta 1054:267-84.
Pinna, L.A. (1994) A historical view of protein kinase CK2. Cell Mol Biol Res 40:383-90.
Pinna, L.A. und Allende, J.E. (2009) Protein kinase CK2 in health and disease: Protein kinase CK2: an ugly duckling in the kinome pond. Cell Mol Life Sci 66:1795-9.
Prowald, K., Fischer, H. und Issinger, O.G. (1984) Enhanced casein kinase II activity in human tumour cell cultures. FEBS Lett 176:479-83.
Prudent, R., Moucadel, V., Laudet, B., Barette, C., Lafanechere, L., Hasenknopf, B., Li, J., Bareyt, S., Lacöte, E., Thorimbert, S., Malacria, M., Gouzerh, P. und Cochet, C. (2008) Identification of polyoxometalates as nanomolar noncompetitive inhibitors of protein kinase CK2. Chem Biol 15:683-92.
Prudent, R., Sautel, C.F. und Cochet, C. (2010) Structure-based discovery of small molecules targeting different surfaces of protein-kinase CK2. Biochim Biophys Acta 1804:493-8.
Ravi, R. und Bedi, A. (2004) NF-kappaB in cancer-a friend turned foe. Drug Resist Updat 7:53-67.
Roig, J., Krehan, A., Colomer, D., Pyerin, W., Itarte, E. und Plana, M. (1999) Multiple forms of protein kinase CK2 present in leukemic cells: in vitro study of its origin by proteolysis. Mol Cell Biochem 191:229-34.
Ruzzene, M., Penzo, D. und Pinna, L.A. (2002) Protein kinase CK2 inhibitor 4,5,6,7-tetrabromobenzotriazole (TBB) induces apoptosis and caspase-dependent degradation of haematopoietic lineage cell-specific protein 1 (HS1) in Jurkat cells. Biochem J 364:41-7.
Ruzzene, M. und Pinna, L.A. (2010) Addiction to protein kinase CK2: A common denominator of diverse cancer cells? Biochim Biophys Acta 1804:499-504.
Ryu, M.Y., Kim, D.W., Arima, K., Mouradian, M.M., Kim, S.U. und Lee, G. (2008) Localization of CKII beta subunits in Lewy bodies of Parkinson's disease. J Neurol Sci 266:9-12.
Sale, E.M. und Sale, G.J. (2008) Protein kinase B: signalling roles and therapeutic targeting. Cell Mol Life Sci 65:113-27.
Salomoni, P. und Pandolfi, P.P. (2002) The role of PML in tumor suppression. Cell 108:165-70.
Salvi, M., Sarno, S., Cesaro, L., Nakamura, H. und Pinna, L.A. (2009) Extraordinary pleiotropy of protein kinase CK2 revealed by weblogo phosphoproteome analysis. Biochim Biophys Acta 1793:847-59.
Salvi, M., Sarno, S., Marin, O., Meggio, F., Itarte, E. und Pinna, L.A. (2006) Discrimination between the activity of protein kinase CK2 holoenzyme and its catalytic subunits. FEBS Lett 580:3948-52.
Sarno, S., De Moliner, E., Ruzzene, M., Pagano, M.A., Battistutta, R., Bain, J., Fabbro, D., Schoepfer, J., Elliott, M., Furet, P., Meggio, F., Zanotti, G. und Pinna, L.A. (2003) Biochemical and three-dimensional-structural study of the specific inhibition of protein kinase CK2 by [5-oxo-5,6-dihydroindolo-(1,2-a)quinazolin-7-yl]acetic acid (IQA). Biochem J 374:639-46.
Sarno, S., Reddy, H., Meggio, F., Ruzzene, M., Davies, S.P., Donella-Deana, A., Shugar, D. und Pinna, L.A. (2001) Selectivity of 4,5,6,7-tetrabromobenzotriazole, an ATP sitedirected inhibitor of protein kinase CK2 ('casein kinase-2'). FEBS Lett 496:44-8.
Sarno, S., Ruzzene, M., Frascella, P., Pagano, M.A., Meggio, F., Zambon, A., Mazzorana, M., Di Maira, G., Lucchini, V. und Pinna, L.A. (2005) Development and exploitation of CK2 inhibitors. Mol Cell Biochem 274:69-76.
Sato, S., Fujita, N. und Tsuruo, T. (2000) Modulation of Akt kinase activity by binding to Hsp90. Proc Natl Acad Sci U S A 97:10832-7.
Scaglioni, P.P., Yung, T.M., Choi, S.C., Baldini, C., Konstantinidou, G. und Pandolfi, P.P. (2008) CK2 mediates phosphorylation and ubiquitin-mediated degradation of the PML tumor suppressor. Mol Cell Biochem 316:149-54.
Schneider, C.C., Hessenauer, A., Götz, C. und Montenarh, M. (2009) DMAT, an inhibitor of protein kinase CK2 induces reactive oxygen species and DNA double strand breaks. Oncol Rep 21:1593-7.
Schneider, C.C., Hessenauer, A., Montenarh, M. und Götz, C. (2010) p53 is dispensable for the induction of apoptosis after inhibition of protein kinase CK2. Prostate 70:126-34.
Seldin, D.C., Landesman-Bollag, E., Farago, M., Currier, N., Lou, D. und Dominguez, I. (2005) CK2 as a positive regulator of Wnt signalling and tumourigenesis. Mol Cell Biochem 274:63-7.
Seldin, D.C. und Leder, P. (1995) Casein kinase II alpha transgene-induced murine lymphoma: relation to theileriosis in cattle. Science 267:894-7.
Seldin, D.C., Lou, D.Y., Toselli, P., Landesman-Bollag, E. und Dominguez, I. (2008) Gene targeting of CK2 catalytic subunits. Mol Cell Biochem 316:141-7.
Shi, X., Potvin, B., Huang, T., Hilgard, P., Spray, D.C., Suadicani, S.O., Wolkoff, A.W., Stanley, P. und Stockert, R.J. (2001) A novel casein kinase 2 alpha-subunit regulates membrane protein traffic in the human hepatoma cell line HuH-7. J Biol Chem 276:2075-82.
Shimoyama, Y., Sakamoto, R., Akaboshi, T., Tanaka, M. und Ohtsuki, K. (2001) Characterization of secretory type IIA phospholipase A2 (sPLA2-IIA) as a glycyrrhizin (GL)-binding protein and the GL-induced inhibition of the CK-IImediated stimulation of sPLA2-IIA activity in vitro. Biol Pharm Bull 24:1004-8.
Shin, S., Lee, Y., Kim, W., Ko, H., Choi, H. und Kim, K. (2005) Caspase-2 primes cancer cells for TRAIL-mediated apoptosis by processing procaspase-8. EMBO J 24:3532-42.
Siemer, S., Stalter, G., Ziegler, M. und Issinger, O. (1996) Charakterisierung der Proteinkinase CK2 in menschlichen Nierentumoren. Aktuel Urol 27:1-5.
Singh, N.N. und Ramji, D.P. (2008) Protein kinase CK2, an important regulator of the inflammatory response? J Mol Med 86:887-97.
Slaton, J.W., Unger, G., Sloper, D., Davis, A. und Ahmed, K. (2004) Induction of apoptosis by antisense CK2 in human prostate cancer xenograft model. Mol Cancer Res 2:712-21.
Stalter, G., Siemer, S., Becht, E., Ziegler, M., Remberger, K. und Issinger, O.-G. (1994) Asymmetric expression of protein kinase CK2 subunits in human kidney tumors. Biochem Biophys Res Commun 202:141-7.
Sugano, S., Andronis, C., Ong, M.S., Green, R.M. und Tobin, E.M. (1999) The protein kinase CK2 is involved in regulation of circadian rhythms in Arabidopsis. Proc Natl Acad Sci U S A 96:12362-6.
Suzuki, Y., Cluzeau, J., Hara, T., Hirasawa, A., Tsujimoto, G., Oishi, S., Ohno, H. und Fujii, N. (2008) Structure-activity relationships of pyrazine-based CK2 inhibitors: synthesis and evaluation of 2,6-disubstituted pyrazines and 4,6-disubstituted pyrimidines. Arch Pharm (Weinheim) 341:554-61.
Tawfic, S., Yu, S., Wang, H., Faust, R., Davis, A. und Ahmed, K. (2001) Protein kinase CK2 signal in neoplasia. Histol Histopathol 16:573-82.
Thornburg, W. und Lindell, T.J. (1977) Purification of rat liver nuclear protein kinase NII. J Biol Chem 252:6660-5.
Tiganis, T., House, C.M. und Kemp, B.E. (1993) Casein kinase II beta-subunit inhibits the activity of the catalytic alpha-subunit in the absence of salt. Biochim Biophys Acta 1203:282-9.
Torres, J. und Pulido, R. (2001) The tumor suppressor PTEN is phosphorylated by the protein kinase CK2 at its C terminus. Implications for PTEN stability to proteasome-mediated degradation. J Biol Chem 276:993-8.
Trembley, J.H., Wang, G., Unger, G., Slaton, J. und Ahmed, K. (2009) Protein kinase CK2 in health and disease: CK2: a key player in cancer biology. Cell Mol Life Sci 66:1858-67.
Tsuchiya, Y., Akashi, M., Matsuda, M., Goto, K., Miyata, Y., Node, K. und Nishida, E. (2009) Involvement of the protein kinase CK2 in the regulation of mammalian circadian rhythms. Sci Signal 2:ra26.
Ubersax, J.A. und Ferrell, J.E. (2007) Mechanisms of specificity in protein phosphorylation. Nat Rev Mol Cell Biol 8:530-41.
Unger, G., Davis, A.T., Slaton, J.W. und Ahmed, K. (2004) Protein kinase CK2 as regulator of cell survival: implications for cancer therapy. Curr Cancer Drug Tar 4:77-84.
Valero, E., De Bonis, S., Filhol, O., Wade, R.H., Langowski, J., Chambaz, E.M. und Cochet, C. (1995) Quaternary structure of casein kinase 2. Characterization of multiple oligomeric states and relation with its catalytic activity. J Biol Chem 270:8345-52.
Vilk, G., Weber, J.E., Turowec, J.P., Duncan, J.S., Wu, C., Derksen, D.R., Zien, P., Sarno, S., Donella-Deana, A., Lajoie, G., Pinna, L.A., Li, S.S.C. und Litchfield, D.W. (2008) Protein kinase CK2 catalyzes tyrosine phosphorylation in mammalian cells. Cell Signal 20:1942-51.
Villar-Palasi, C. und Kumon, A. (1981) Purification and properties of dog cardiac troponin T kinase. J Biol Chem 256:7409-15.
Walsh, D.A., Perkins, J.P. und Krebs, E.G. (1968) An adenosine 3',5'-monophosphatedependant protein kinase from rabbit skeletal muscle. J Biol Chem 243:3763-5.
Wang, G., Unger, G., Ahmad, K., Slaton, J.W. und Ahmed, K. (2005) Downregulation of CK2 induces apoptosis in cancer cells-a potential approach to cancer therapy. Mol Cell Biochem 274:77-84.
Wang, L., Lin, L. und Ye, B. (2006) Electrochemical studies of the interaction of the anticancer herbal drug emodin with DNA. J Pharm Biomed Anal 42:625-9.
Wang, S. und Jones, K.A. (2006) CK2 controls the recruitment of Wnt regulators to target genes in vivo. Curr Biol 16:2239-44.
Willert, K., Brink, M., Wodarz, A., Varmus, H. und Nusse, R. (1997) Casein kinase 2 associates with and phosphorylates dishevelled. EMBO J 16:3089-96.
Xu, X., Landesman-Bollag, E., Channavajhala, P.L. und Seldin, D.C. (1999a) Murine protein kinase CK2: gene and oncogene. Mol Cell Biochem 191:65-74.
Xu, X., Toselli, P.A., Russell, L.D. und Seldin, D.C. (1999b) Globozoospermia in mice lacking the casein kinase II alpha' catalytic subunit. Nat Genet 23:118-21.
Yamada, M., Katsuma, S., Adachi, T., Hirasawa, A., Shiojima, S., Kadowaki, T., Okuno, Y., Koshimizu, T.-a., Fujii, S., Sekiya, Y., Miyamoto, Y., Tamura, M., Yumura, W., Nihei, H., Kobayashi, M. und Tsujimoto, G. (2005) Inhibition of protein kinase CK2 prevents the progression of glomerulonephritis. Proc Natl Acad Sci U S A 102:7736-41.
Yan, J.X., Packer, N.H., Gooley, A.A. und Williams, K.L. (1998) Protein phosphorylation: technologies for the identification of phosphoamino acids. J Chromatogr A 808:23-41.
Yang, Y., Cheng, P. und Liu, Y. (2002) Regulation of the Neurospora circadian clock by casein kinase II. Genes Dev 16:994-1006.
Yenice, S., Davis, A.T., Goueli, S.A., Akdas, A., Limas, C. und Ahmed, K. (1994) Nuclear casein kinase 2 (CK-2) activity in human normal, benign hyperplastic, and cancerous prostate. Prostate 24:11-6.
Yim, H., Lee, Y.H., Lee, C.H. und Lee, S.K. (1999) Emodin, an anthraquinone derivative isolated from the rhizomes of Rheum palmatum, selectively inhibits the activity of casein kinase II as a competitive inhibitor. Planta Med 65:9-13.
Zandomeni, R., Zandomeni, M.C., Shugar, D. und Weinmann, R. (1986) Casein kinase type II is involved in the inhibition by 5,6-dichloro-1-beta-D-ribofuranosylbenzimidazole of specific RNA polymerase II transcription. J Biol Chem 261:3414-9.
Zien, P., Duncan, J.S., Skierski, J., Bretner, M., Litchfield, D.W. und Shugar, D. (2005) Tetrabromobenzotriazole (TBBt) and tetrabromobenzimidazole (TBBz) as selective inhibitors of protein kinase CK2: evaluation of their effects on cells and different molecular forms of human CK2. Biochim Biophys Acta 1754:271-80.

## Patentansprüche

1. Verbindung der allgemeinen Formel I 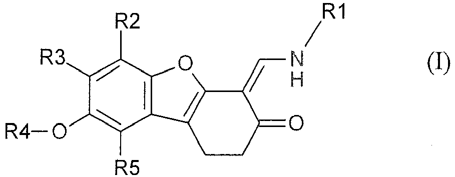 zur Verwendung als Arzneimittel, wobei R1 eine substituierte oder unsubstituierte Arylgruppe oder eine substituierte oder unsubstituierte Alkylgruppe mit 1 bis 16 C-Atomen, vorzugsweise eine Halogen, Alkoxy und/oder Alkyl substituierte Arylgruppe ist; R2, R3 und R5 unabhängig voneinander F, Cl, Br, I, H, eine Hydroxylgruppe oder Alkoxygruppe mit 1 bis 4 C-Atomen sind; und R4 H, eine Alkyl- oder Acylgruppe mit 1 bis 16 C-Atomen, vorzugsweise (CₙH₂ₙ₊₁)-CO mit n=1, 2, 3, 4, 5, 6, 7, 8 ist.

2. Verbindung zur Verwendung als Arzneimittel gemäß Anspruch 1, wobei R1 eine parasubstituierte Arylgruppe ist; R2 und R3 unabhängig voneinander F, Cl oder H sind; R4 H oder CH₃CO ist; und R5 H ist.

3. Verbindung zur Verwendung als Arzneimittel gemäß Anspruch 2, wobei R1 = 4-CH₃OC₆H₄, 4-CH₃C₆H₄ oder 4-FC₆H₆ ist.

4. Verbindung zur Verwendung als Arzneimittel gemäß einem der vorhergehenden Ansprüche, wobei R1 = 4-CH₃C₆H₄; R2 und R3 = Cl; und R4 und R5 = H sind.

5. Verbindung zur Verwendung als Arzneimittel gemäß Anspruch 1, wobei R1 = 4-CH₃OC₆H₄; R3 und R5 = Cl; und R2 und R4 = H sind.

6. Verbindung zur Verwendung als Arzneimittel gemäß einem der vorhergehenden Ansprüche, wobei diese weitere pharmazeutisch gebräuchliche Zusätze und Hilfsstoffe aufweist.

7. Verbindung zur Verwendung als Arzneimittel gemäß einem der vorhergehenden Ansprüche, wobei diese wenigstens eine weitere antineoplastisch wirkende Verbindung aufweist.

8. Verbindung der allgemeinen Formel I 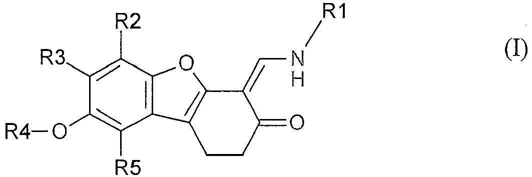 wobei R1 eine substituierte oder unsubstituierte Arylgruppe oder eine substituierte oder unsubstituierte Alkylgruppe mit 1 bis 16 C-Atomen, vorzugsweise eine Halogen, Alkoxy und/oder Alkyl substituierte Arylgruppe ist; R2, R3 und R5 unabhängig voneinander F, Cl, Br, I, H, eine Hydroxylgruppe oder Alkoxygruppe mit 1 bis 4 C-Atomen sind; und R4 H, eine Alkyl- oder Acylgruppe mit 1 bis 16 C-Atomen, vorzugsweise (CₙH₂ₙ₊₁)-CO mit n=1, 2, 3, 4, 5, 6, 7, 8 ist, zur Verwendung in der Behandlung neoplastischer Erkrankungen.

## Claims

1. A compound according to the, general formula I: 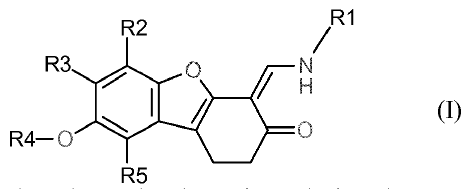 for use as pharmaceutical product, wherein R1 is a substituted or unsubstituted aryl group or a substituted or unsubstituted alkyl group containing 1 to 16 C atoms, preferably a halogen, alkoxy and/or alkyl-substituted aryl group; R2, R3 and R5 are independently F, Cl, Br, I, H, a hydroxyl group or an alkoxy group containing 1 to 4 C atoms; and R4 is H, an alkyl or acyl group containing 1 to 16 C atoms, preferably (CₙH₂ₙ₊₁)-CO, with n=1, 2, 3, 4, 5, 6, 7 or 8.

2. The compound for use as a pharmaceutical product according to claim 1, wherein R1 is a para-substituted aryl group; R2 and R3 are independently F, Cl or H; R4 is H or CH₃CO; and R5 is H.

3. The compound for use as a pharmaceutical product according to claim 2, wherein R1 = 4-CH₃OC₆H₄, 4-CH₃C₆H₄ or 4-FC₆H₆.

4. The compound for use as a pharmaceutical product according to one of the preceding claims, wherein R1 = 4-CH₃C₆H₄; R2 and R3 = Cl; and R4 and R5 = H.

5. The compound for use as a pharmaceutical product according to claim 1, wherein R1 = 4-CH₃OC₆H₄; R3 and R5 = Cl; and R2 and R4 = H.

6. The compound for use as a pharmaceutical product according to one of the preceding claims, further comprising pharmaceutically acceptable additives and auxiliary substances.

7. The compound for use as a pharmaceutical product according to one of the preceding claims, comprising at least one further compound with an anti-neoplastic effect.

8. Compound with general formula I 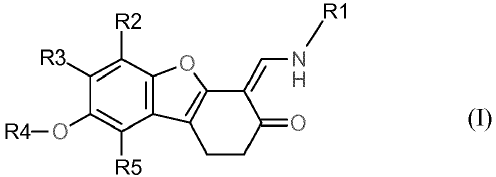 wherein R1 is a substituted or unsubstituted aryl group or a substituted or unsubstituted alkyl group containing 1 to 16 C atoms, preferably a halogen, alkoxy and/or alkylsubstituted aryl group; R2, R3 and R5 are independently F, Cl, Br, I, H, a hydroxyl group or an alkoxy group containing 1 to 4 C atoms; and R4 is H, an alkyl or acyl group containing 1 to 16 C atoms, preferably (CₙH₂ₙ₊₁)-CO, with n=1, 2, 3, 4, 5, 6, 7 or 8, for use in treatment of neoplastic diseases.

## Revendications

1. Composé selon la formule générale I 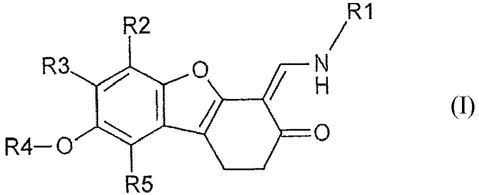 pour l'utilisation en tant que produit pharmaceutique, où R1 est un groupe aryle substitué ou non substitué, ou un groupe alkyle substitué ou non substitué, avec 1 à 16 atomes C, de préférence, un groupe aryle substitué d'un atome d'halogène, d'un groupe alcoxy et/ou d'un groupe alkyle ; R2, R3 et R5 sont indépendamment les uns des autres des atomes F, Cl, Br, I, H, un groupe hydroxyle ou un groupe alcoxy avec 1 à 4 atomes C ; et R4 est un atome H, un groupe alkyle ou acyle avec 1 à 16 atomes C, de préférence, (CₙH₂ₙ₊₁)-CO avec n = 1, 2, 3, 4, 5, 6, 7, 8.

2. Composé pour l'utilisation en tant que produit pharmaceutique selon la revendication 1, dans lequel R1 est un groupe aryle substitué en para ; R2 et R3 sont indépendamment l'un de l'autre un atome F, Cl ou H ; R4 est un atome H ou un groupe CH₃CO ; et R5 est un atome H.

3. Composé pour l'utilisation en tant que produit pharmaceutique selon la revendication 2, dans lequel R1 = 4-CH₃OC₆H₄, 4-CH₃C₆H₄ ou 4-FC₆H₆.

4. Composé pour l'utilisation en tant que produit pharmaceutique selon l'une des revendications précédentes, dans lequel R1 est = 4-CH₃C₆H₄ ; R2 et R3 sont = Cl ; et R4 et R5 sont = H.

5. Composé pour l'utilisation en tant que produit pharmaceutique selon la revendication 1, dans lequel R1 est = 4-CH₃OC₆H₄, R3 et R5 sont = CL ; et R2 et R4 sont = H.

6. Composé pour l'utilisation en tant que produit pharmaceutique selon l'une des revendications précédentes, dans lequel celui-ci présente d'autres additifs et produits auxiliaires pharmaceutiquement acceptables.

7. Composé pour l'utilisation en tant que produit pharmaceutique selon l'une des revendications précédentes, dans lequel celui-ci présente au moins un autre composé agissant comme antinéoplasique.

8. Composé selon la formule générale I 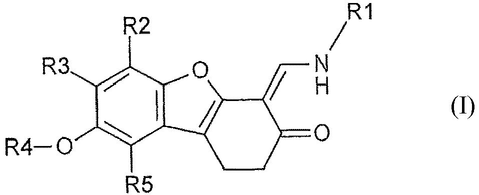 où R1 est un groupe aryle substitué ou non substitué, ou un groupe alkyle substitué ou non substitué, avec 1 à 16 atomes C, de préférence, un groupe aryle substitué d'un atome d'halogène, d'un groupe alcoxy et/ou d'un groupe alkyle ; R2, R3 et R5 sont indépendamment les uns des autres des atomes F, Cl, Br, I, H, un groupe hydroxyle ou un groupe alcoxy avec 1 à 4 atomes C ; et R4 est un atome H, un groupe alkyle ou acyle avec 1 à 16 atomes C, de préférence, (CₙH₂ₙ₊₁)-CO avec n = 1, 2, 3, 4, 5, 6, 7, 8, pour l'utilisation dans le traitement de maladies néoplasiques.
